# FASCICULE DE BREVET EUROPEEN

(11) **EP 0 566 445 B1**
(45) Date de publication et mention de la délivrance du brevet: **07.02.1996**
(21) Numéro de dépôt: 93400840.0
(22) Date de dépôt: 01.04.1993
(51) Int. Cl.: C07C 281/12, C07C 337/08, C07C 281/06, A61K 31/175

(54) **Dérivés d'indane-1,3-dione et d'indane-1,2,3-trione, leurs procédés de préparation et leur application en thérapeutique**
Indan-1,3-Dion und Indan-1,2,3-Trionderivate, Verfahren zu ihrer Herstellung und ihre therapeutische Verwendung
Derivatives of indane 1,3-dione and indane-1,2,3-trione, processes for their preparation and their application in therapy

(30) Priorité: 03.04.1992 FR 9204071
(43) Date de publication de la demande: 20.10.1993
(73) Titulaire: INNOTHERA, F-94110 Arcueil (FR)
(72) Inventeur: Cugnon de Sevricourt, Michel, F-14370 Moult (FR); Dacquet, Catherine, F-75005 Paris (FR); Finet, Michel, F-94260 Fresnes (FR); Le Marquer, Florence, F-75005 Paris (FR); Robba, Max, F-75004 Paris (FR); Tembo, Norbert Olivier, F-95800 Cergy Saint Christophe (FR); Yannic-Arnoult, Sylvie, F-91350 Grigny (FR); Torregrosa, Jean-Luc, F-93200 Saint Denis (FR)
(74) Mandataire: Kedinger, Jean-Paul

(56) Documents cités:
- EP-A- 0 172 128
- EP-A- 0 236 151
- EP-A- 0 456 133
- DE-A- 2 127 982
- US-A- 3 280 185
- JOURNAL OF ORGANIC CHEMISTRY. vol. 38, no. 12, 15 Juin 1973, EASTON US pages 2251 - 2 D. BEN-ISHAI ET. AL. 'Rearrangement in the indan-1,3-dione system'
- SYNTHESIS. no. 3, Mars 1973, STUTTGART DE pages 154 - 5 D MATTHIES ET. AL. 'Amidoalkierende Derivate des Ninhydrins'
- JOURNAL OF PHARMACEUTICAL SCIENCES vol. 56, no. 6, Juin 1967, WASHINGTON US pages 775 - 6 R.S. VARMA ET. AL. 'Thiosemicarbazones derived from indanedione- 1,3'
- CHEMICAL ABSTRACTS, vol. 72, no. 11, 16 Mars 1970, Columbus, Ohio, US; abstract no. 55046r, A. VEVERIS ET. AL. 'New derivatives of 1,3-indandione thiosemicarbazone' page 384 ;colonne 1 ;
- CHEMICAL ABSTRACTS, vol. 83, no. 1, 7 Juillet 1975, Columbus, Ohio, US; abstract no. 9584q, L. GEITA ET. AL. '2-Acetamido-1,3-indandione and its derivatives' page 793 ;colonne 1 ;
- CHEMICAL ABSTRACTS, vol. 81, no. 19, 11 Novembre 1974, Columbus, Ohio, US; abstract no. 120286s, P.A. CROOKS 'New Synthesis of 2-Aminoindanes' page 503; colonne 1;
- CHEMICAL ABSTRACTS, vol. 67, no. 23, 4 Décembre 1967, Columbus, Ohio, US; abstract no. 107890a, M. FRIEDMAN 'Mechanism of the ninhydrin reaction. II. Preparation and spectral properties of reaction products from primary aromatic amines and ninhydrin hydrate' page 10155 ;colonne 1 ;
- CHEMICAL ABSTRACTS, vol. 84, no. 7, 16 Février 1976, Columbus, Ohio, US; abstract no. 43946q, S. SCHNEPP ET. AL. 'Oxidation of semicarbazones with selenium dioxide' page 484 ;colonne 2 ;

## Description

La présente invention a pour objet de nouveaux dérivés de l'indane-1,2-dione et de l'indane-1,2,3-trione, leurs procédés de préparation et leur application en thérapeutique.

Ces dérivés répondent plus précisément à la formule : 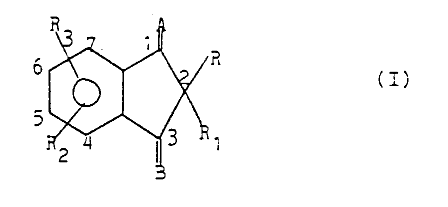
dans laquelle R₂ et R₃ représentent indépendamment l'un de l'autre H, alkoxy en C₁-C₄ ou OH ; et le couple (A, B) prend la valeur :
- (oxygène, oxygène), auquel cas l'un parmi R et R₁ représente OH, halogène, (alkyl en C₁-C₄)NH, N-morpholino(alkyl en C₁-C₄)NH, 1-(pyridyl)-4-pipérazino ou 1-(phényl)-4-pipérazino dont le noyau phényle est éventuellement substitué par un halogène et l'autre représente un groupe NHNHCONHR₄ où R₄ = phényle ou phényle substitué par OH ou alkyle en C₁-C₄.
   R et R₁ pouvant par ailleurs former ensemble un groupe :
   . =N-NH-CX-NHR₅ où X représente oxygène ou soufre et R₅ = H, pyridyle, phényle, ou phényle substitué par un, deux ou trois groupes choisis parmi OH, CF₃, alkyle en C₁-C₄, alkoxy en C₁-C₄, halogène, méthylènedioxy, acétoxy et hydroxyéthyle,
   . =NNHCXN(phényle)₂, où X représente oxygène ou soufre,
   . =N-NH-CX-NH-NH-R₅ où X et R₅ ont les mêmes significations que ci-dessus, ou
   . =N-NH-C(SCH₃)=NR₆ ou =N-N=C(SCH₃)-NHR₆ où R₆ représente phényle ou phényle substitué par un, deux ou trois groupes choisis parmi OH, CF₃, alkyle en C₁-C₄, alkoxy en C₁-C₄, halogène, méthylènedioxy, acétoxy et hydroxyéthyle ;
- (N-OH, oxygène), auquel cas R et R₁ forment ensemble un groupe =NNHCXNHR₅ ou =NNHCXN(phényle)₂ où X et R₅ ont les mêmes significations que ci-dessus ;
- (NNHCXNHR₅, oxygène) où X et R₅ ont les mêmes significations que ci-dessus, auquel cas R et R₁ forment ensemble un groupe =NNHCXNHR₅ ; ou
- (N-OH, N-OH), auquel cas R et R₁ forment ensemble un groupe =NNHCXNHR₅ où X et R₅ ont les mêmes significations que ci-dessus ou un groupe =N-NH-CX-N(phényle)₂.

Entrent dans la portée de la formule (I) ci-dessus :
(i) les composés de formule : 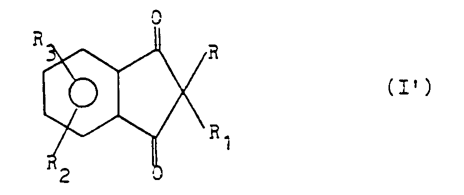 où R, R₁, R₂ et R₃ ont les mêmes significations que dans la formule (I) quand le couple (A, B) y prend la valeur (oxygène, oxygène),
(ii) les composés de formule : 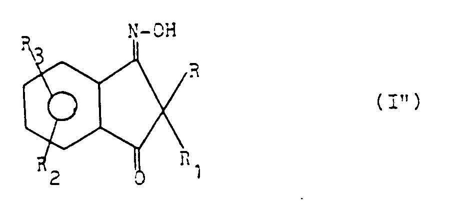 où R, R₁, R₂ et R₃ ont les mêmes significations que dans la formule (I) quand le couple (A, B) y prend la valeur (=N-OH, oxygène),
(iii) les composés de formule : 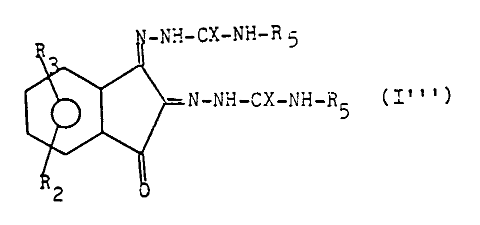 où R₂, R₃, X et R₅ ont les mêmes significations que dans la formule (I), et
(iv) les composés de formule : 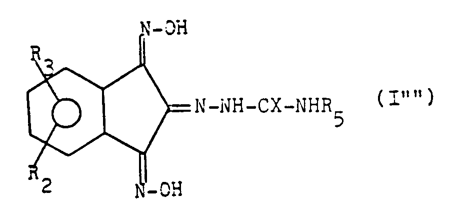 où R₂, R₃, X et R₅ ont les mêmes significations que dans la formule (I).

Entrent dans la portée de la formule (I) ci-dessus :
(a) les composés de formule : 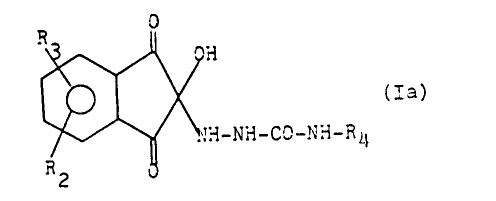
(b) les composés de formule : 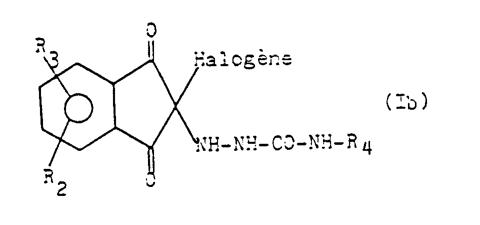
(c) les composés de formule : 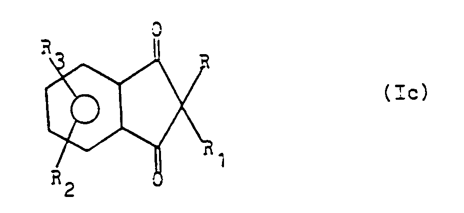 où l'un parmi R et R₁ représente NHNHCONHR₄ et l'autre représente (alkyl en C₁-C₄)NH, N-morpholino(alkyl en C₁-C₄)NH, 1-(pyridyl)-4-pipérazino ou 1-(phényl)-4-pipérazino dont le noyau phényle est éventuellement substitué par un halogène.
(d) les composés de formule : 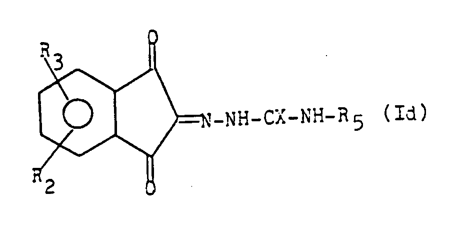
(e) les composés de formule : 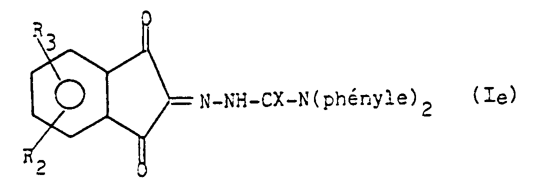
(f) les composés de formule : 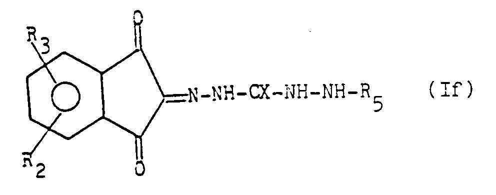
(g) les composés de formule : 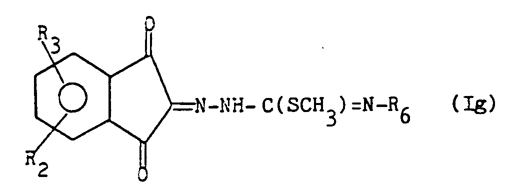
(h) les composés de formule : 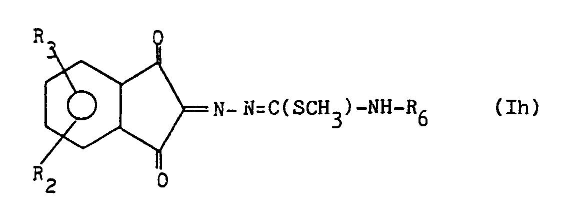
(i) les composés de formule : 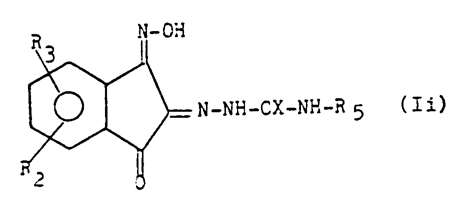
(j) les composés de formule : 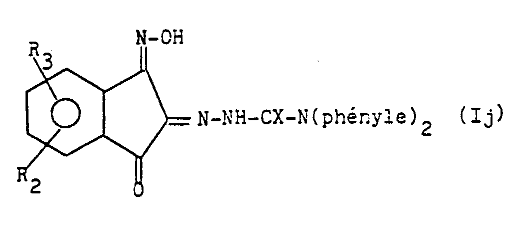
(k) les composés de formule : 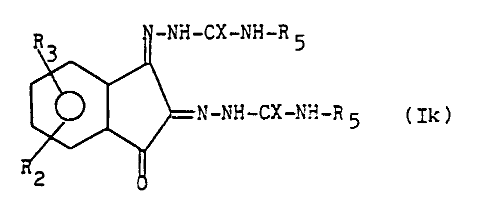
(l) les composés de formule : 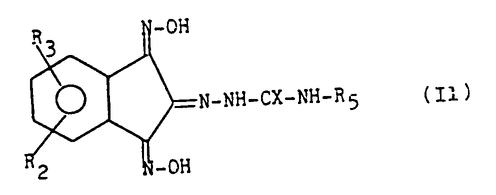
(m) les composés de formule : 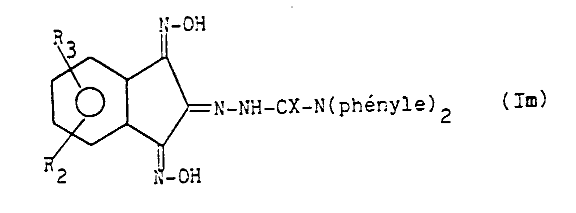 les symboles R₂, R₃, R₄, R₅ et X ayant les mêmes significations que dans la formule (I).

Dans les formules (I') à (I'''') et (Ia) à (Im) ci-dessus, R₂ est notamment en position 5 et R₃ en position 6.

On ajoutera également que dans les formules (I') à (I'''') et (Ia) à (Im), le couple (R₂, R₃) peut notamment être (H, H), (5-OCH₃,H), (5-OH, H) ou (5-OCH3, 6-OCH₃).

La présente invention comprend également les sels des composés salifiables parmi ceux décrits ci-dessus. Ces sels comprennent les sels d'addition d'acides minéraux tels que l'acide chlorhydrique, bromhydrique, sulfurique ou phosphorique, et les sels d'addition d'acides organiques tels que l'acide acétique, propionique, oxalique ou citrique.

L'invention englobe en outre tous les stéréoisoméres possibles des dérivés de formule (I) et les mélanges de tels stéréoisomères, ainsi que les métabolites de ces dérivés.

Entrent également dans la portée de la présente invention, les procédés de préparation des dérivés de formule (I). Ces procédés font l'objet des schémas 1 à 6 ci-après dans lesquels les symboles R à R₆ et X ont, sauf autre indication, les mêmes significations que dans la formule (I).

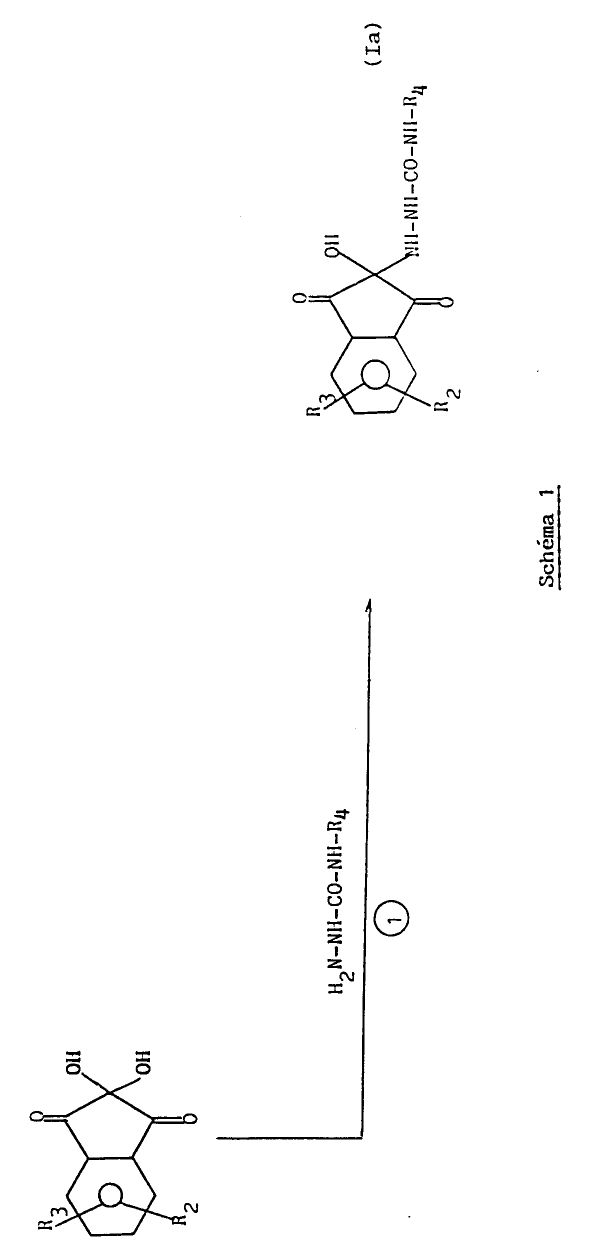

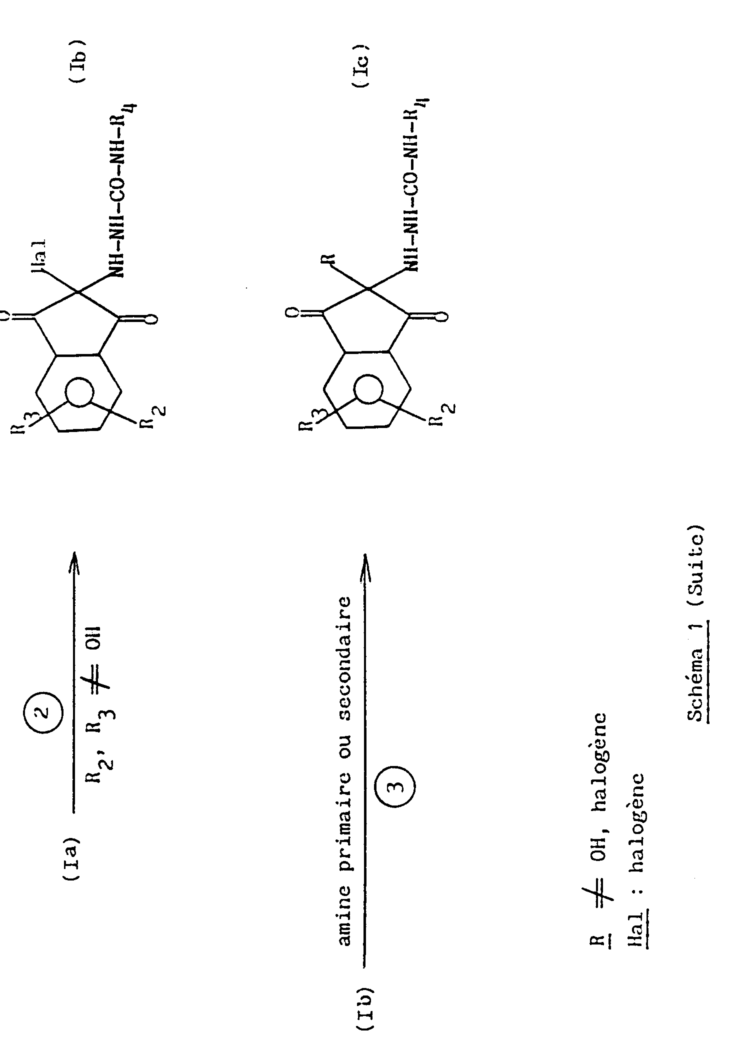

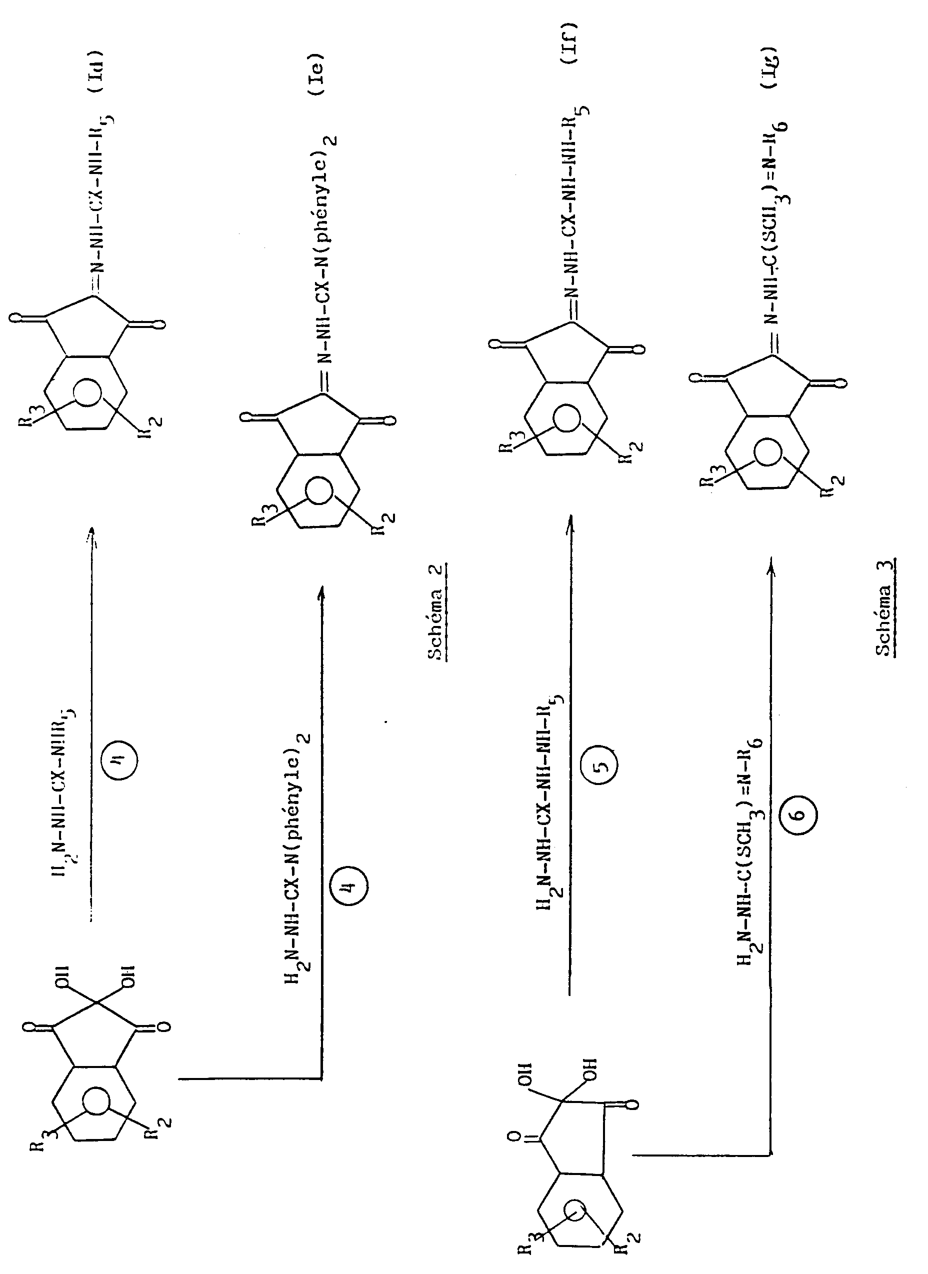

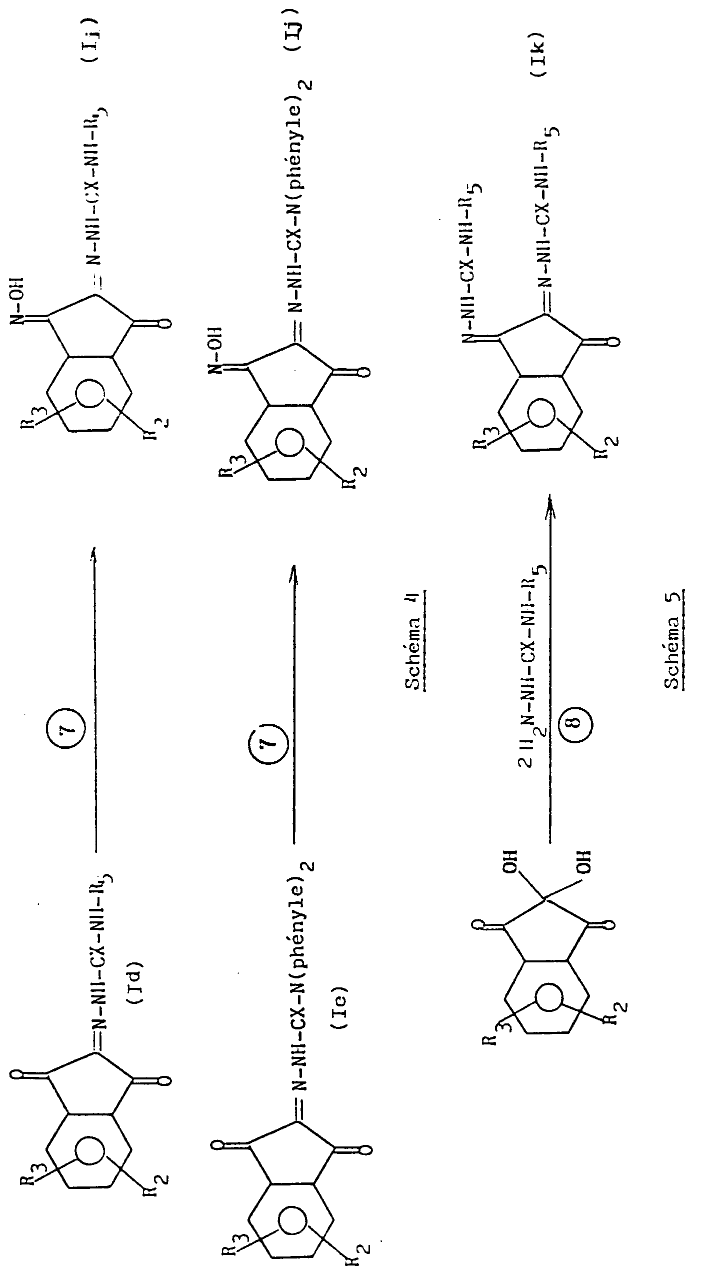

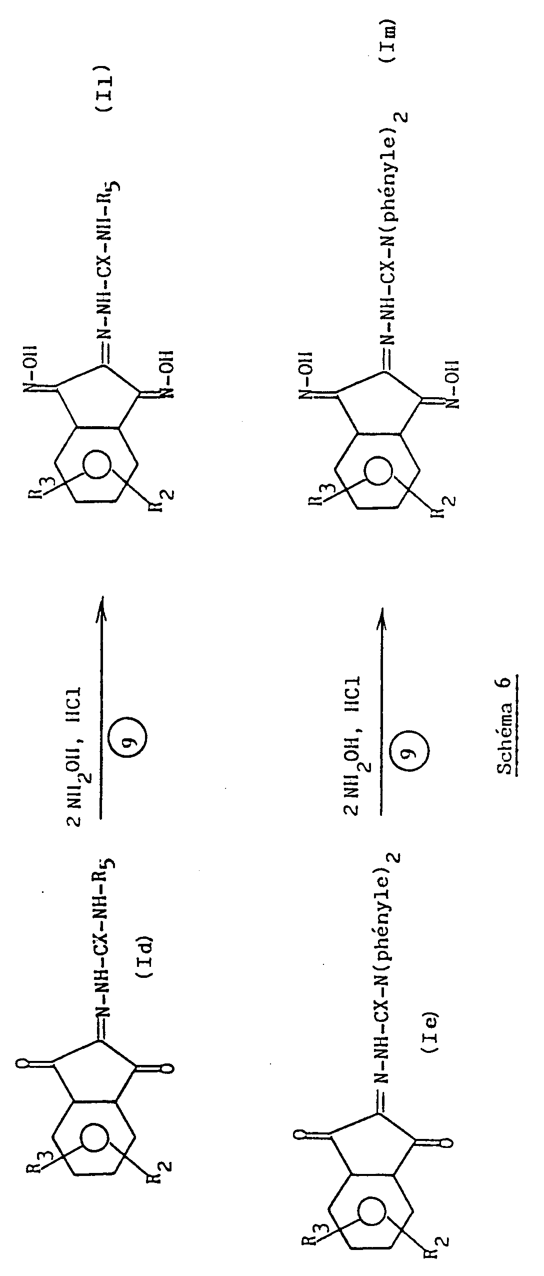

Dans les schémas ci-dessus, ① à ⑨ désignent les méthodes utilisées et ont les significations suivantes :
① Condensation dans un solvant tel que l'éthanol, à chaud, de préférence au reflux.
② Réaction avec un agent halogénant, notamment un agent chlorant et de préférence le chlorure de thionyle, dans un solvant tel que le THF, à chaud, de préférence au reflux.
③ Condensation avec une amine primaire [(alkyle en C₁-C₄)NH₂ ou N-morpholino(alkyl en C₁-C₄)NH₂] ou une amine secondaire [1-(pyridyl)-4-pipérazine ou 1-(phényl)-4- pipérazine dont le noyau phényle est éventuellement substitué par un atome d'halogène], dans un solvant tel que l'éther diéthylique, en présence d'une base telle que la triéthylamine.
④ Condensation du semicarbazide ou thiosemicarbazide, sous forme de chlorhydrate, dans un solvant tel qu'un mélange éthanol-eau, à chaud, de préférence au reflux, ou Condensation du semicarbazide ou thiosemicarbazide, dans un solvant tel que l'éthanol, de préférence à température ambiante.
⑤ Condensation dans un solvant, notamment une solution aqueuse d'éthanol, de préférence à température ambiante, suivie du traitement du produit de réaction par HCl dans l'éthanol.
⑥ Condensation dans un solvant, notamment une solution aqueuse d'éthanol, à chaud. On précisera ici que les composés de formule (Ig) peuvent également exister sous la forme tautomère (Ih).
⑦ Condensation avec le chlorhydrate d'hydroxylamine, en présence d'acétate de sodium, dans un solvant tel que l'éthanol aqueux, à chaud et de préférence au reflux.
⑧ Mêmes conditions réactionnelles que dans ④ mais utilisation de deux équivalents de semicarbazide ou thiosemicarbazide.
⑨ Condensation à chaud, de préférence au reflux, dans un solvant tel que l'éthanol aqueux, en présence d'acétate de sodium.

Les préparations suivantes sont données à titre d'exemples pour illustrer l'invention.

### A/ Préparation des matières premières

### Exemple a

### 5-Hydroxyindane-1,3-dione

A une suspension de 0,01 mole de 5-acétoxy-2-carbéthoxy-3-hydroxy-1-indanone dans 80 ml d'eau,on ajoute 4 ml d'acide sulfurique 10N.Le mélange réactionnel est porté au reflux pendant 15 minutes.L'insoluble est filtré à chaud et le précipité jaune formé après refroidissement est essoré,lavé à l'eau glacée puis séché.
- Rdt. :: 85 %
- F :: 208°C
- IR :: ν OH = 3250 cm⁻¹
ν CO = 1730 et 1680 cm⁻¹

### Exemple b

### 2-Bromo-5-hydroxyindane-1,3-dione

A une solution de 0,02 mole de 5-hydroxyindane-1,3-dione dans 60 ml de chloroforme,on ajoute goutte à goutte 0,02 mole de brome.Le mélange réactionnel est agité à 50°C pendant 30 minutes et le précipité obtenu après évaporation du solvant est recristallisé
- Rdt :: 60 %
- F :: 194°C
- IR :: ν OH = 3380 cm⁻¹
ν CO = 1740 et 1710 cm⁻¹
- solvt. de recristallisation :: mélange (V/V) éther éthylique/éther de pétrole

### Exemple c

### 5-Hydroxyindane-1,2,3-trione,monohydrate

Une solution de 0,01 mole de 2-bromo-5-hydroxyindane-1,3-dione dans 10 ml de diméthylsulfoxyde est chauffée à 80°C pendant 30 minutes.On ajoute 50 ml d'une solution d'acide chlorhydrique 1N, puis on prolonge le chauffage de 30 minutes.Après refroidissement,la suspension huileuse obtenue est extraite à l'éther éthylique.La phase organique est lavée à l'eau et séchée au sulfate de magnésium.

Le précipité jaune obtenu après évaporation du solvant est recristallisé dans l'eau.
- Rdt :: 45 %
- F :: > 265°C
- IR :: ν OH = 3340 cm⁻¹
ν CO = 1750 et 1710 cm⁻¹

### Exemple d

### 5,6-Diméthoxyindane-1,2,3-trione,monohydrate

A une solution de 0,03 mole de 5,6-diméthoxy-1-indanone dans 100 ml de dioxanne,on ajoute 0.06 mole d'oxyde de sélénium en solution dans 2,5 ml d'eau.Le mélange réactionnel est porté au reflux pendant 7 heures et le résidu obtenu après évaporation du solvant est dissous dans l'acétate d'éthyle.La phase organique est lavée plusieurs fois à l'eau,séchée sur sulfate de magnésium puis décolorée.

Le précipité obtenu après évaporation du solvant est recristallisé dans l'éther éthylique.
- Rdt :: 50 %
- F :: 170°C
- IR :: ν CO = 1730 et 1700 cm⁻¹

### Exemple e

### 6-Hydroxy-5-méthoxyindane-1,2,3-trione,monohydrate

A une solution de 0,03 mole de 6-hydroxy-5-méthoxy-1-indanone dans 100 ml de dioxane,on ajoute 0,06 mole d'oxyde de sélénium en solution dans 2,5 ml d'eau.Le mélange réactionnel est porté au reflux pendant 7 heures et le résidu obtenu après évaporation du solvant est dissous dans l'acétate d'éthyle.La phase organique est lavée plusieurs fois à l'eau,séchée sur sulfate de magnésium puis décolorée.

Le précipité obtenu après évaporation du solvant est recristallisé dans l'eau.
- Rdt :: 50 %
- F :: > 265°C
- IR :: ν CO = 1740 et 1700 cm⁻¹

### Exemple f

### 2-Oximino-5-hydroxyindane-1,3-dione

A une suspension de 0,01 mole de 5-hydroxyindane-1,3-dione dans 6 ml d'une solution d'acide sulfurique 2N,on ajoute goutte à goutte en maintenant la température à 5°C, 0,02 mole de nitrite de sodium en solution dans 8 ml d'eau.Le mélange réactionnel est agité 4 heures à 5°C.L'insoluble est essoré,lavé à l'eau,séché et recristallisé dans l'acétone.
- Rdt :: 65 %
- F :: 220°C
- IR :: ν OH = 3400 et 3260 cm⁻¹
ν CO = 1730 et 1690 cm⁻¹
Dans ce qui suit, il est fait état de la ninhydrine. Il est rappelé ici que cette dernière est encore connue sous le non d'indane-1,2,3-trione, monohydrate ou sous le nom de 2,2-dihydroxy-1,3-dioxo-2H-indène. Ces trois noms sont utilisés indifféremment dans ce qui précède et dans ce qui suit.

### B/ Préparation des composés de formules (Ia) et (Ib)

### Méthode ①

A une solution de 0,01 mole de ninhydrine substitué ou non dans 20 ml d'éthanol, on ajoute 0,01 mole de H₂N-NH-CO-NH-R₅ en solution dans 20 ml d'éthanol.

Le mélange réactionnel est chauffé à 60° C pendant 5 à 15 minutes et le précipité blanc formé est essoré, lavé à l'éther éthylique puis séché.

### Méthode ②

A une solution de 0,02 mole de composé obtenu par la méthode ① dans 20 ml de tétrahydrofurane,on ajoute goutte à goutte 3,5 ml (0,04 mole) de chlorure de thionyle.Le mélange réactionnel est porté au reflux pendant une heure.L'huile résiduelle obtenue après évaporation de l'excès de chlorure de thionyle et du tétrahydrofurane,cristallise après un lavage à l'éther de pétrole.

### Exemple 1

### 2-Hydroxy-2-[4-(2-hydroxyphényl)-semicarbazido]indane-1,3-dione

### Méthode①

- Matières premières :: Ninhydrine Chlorhydrate de4-(hydroxyphényl)-semicarbazide
- Rdt. :: 90 %
- F :: 190°C
- IR :: ν OH = 3400 cm⁻¹
ν NH = 3210 cm⁻¹
ν CO = 1760, 1720 et 1640 cm⁻¹

### C/ Préparation des composés de formule (Ic)

### Méthode ③

A une solution de 0,007 mole de composé de formule (Ib) dans 60 ml d'éther éthylique,on ajoute 1 ml de triéthylamine.On laisse agiter pendant 5 minutes,puis on ajoute 0,007 mole d'amine.Le mélange réactionnel est agité à température ambiante pendant 2 heures.Le précipité formé est essoré,lavé plusieurs fois à l'eau,séché et recristallisé.

### D/ Préparation des composés de formules (Id) et (Ie)

### Méthode ④

A une solution de 0,01 mole de ninhydrine substituée ou non, dans 50 ml d'ethanol, on ajoute 0,01 mole de chlorhydrate de semicarbazide(ou thiosemicarbazide) correspondant en solution dans 10 ml d'eau.

Le mélange réactionnel est porté au reflux pendant 1 heure(ou 30min. pour les exemples 11 à 14). Le précipité formé est essoré à chaud (ou à froid pour les exemples 12 à 15),séché et recristallisé.

Dans le cas où le précipité formé est un mélange des composés mono et disubstitué la séparation se fait par recristallisation fractionnée.

### Exemple 2

### 2-Semicarbazono-indane-1,3-dione

- Matières premières :: Ninhydrine Chlorhydrate de semicarbazide
- Rdt :: 70 %
- F :: 255°C
- IR :: ν NH₂ et NH = 3450,3350 et 3220 cm⁻¹
ν CO = 1720 et 1680 cm⁻¹
- solvt. de recristallisation :: acétonitrile

### Exemple 3

### 2-(4-Phénylsemicarbazono)-indane-1,3-dione

- Matières premières :: Ninhydrine Chlorhydrate de 4-phénylsemicarbazide
- Rdt :: 40 %
- F :: 220°C
- IR :: ν NH = 3350 et 3250 cm⁻¹
ν CO = 1730,1715 et 1680 cm⁻¹
- solvt. de recristallisation :: acétate d'éthyle

### Exemple 4

### 2-[4-(2-hydroxyphényl)-semicarbazono]indane-1,3-dione

- Matières premières :: Ninhydrine Chlorhydrate de 4-(2-hydroxyphényl)-semicarbazide
- Rdt :: 50 %
- F :: 260°C
- IR :: ν OH = 3460 cm⁻¹
ν NH = 3370 et 3250 cm⁻¹
ν CO = 1735 et 1680 cm⁻¹
- solvt. de recristallisation :: éthanol

### Exemple 5

### 2-(4-Phénylthiosemicarbazono)-indane-1,3-dione

- Matières premières :: Ninhydrine Chlorhydrate de 4-phénylthiosemicarbazide
- Rdt. :: 45 %
- F :: 222°C
- IR :: ν NH = 3360 et 3240 cm⁻¹
ν CO = 1730 et 1680 cm⁻¹
- solvt. de recristallisation :: éthanol

### Exemple 6

### 2-[4-(2-Méthoxyphényl)-semicarbazono]indane-1,3-dione

- Matières premières :: Ninhyrine Chlorhydrate de 4-(2-méthoxyphényl)-semicarbazide
- Rdt. :: 80 %
- F :: 235°C
- IR :: ν NH = 3360 et 3240 cm⁻¹
ν CO = 1730 et 1690 cm⁻¹
- solvt. de recristallisation :: méthanol

### Exemple 7

### 2-[4-(3-Méthoxyphényl)-semicarbazono]indane-1,3-dione,monohydrate

- Matières premières :: Ninhydrine Chlorhydrate de 4-(3-méthoxyphényl)-semicarbazide
- Rdt. :: 70 %
- F :: 195°C
- IR :: ν OH = 3600 et 3500 cm⁻¹
ν NH = 3260 cm⁻¹
ν CO = 1725 et 1680 cm⁻¹
- solvt. de recristallisation :: éthanol

### Exemple 8

### 2-[4-(4-Méthoxyphényl)-semicarbazono]indane-1,3-dione

- Matières premières :: Ninhydrine Chlorhydrate de 4-(4-méthoxyphényl)-semicarbazide
- Rdt. :: 80 %
- F :: 218°C
- IR :: ν NH = 3280 et 3220 cm⁻¹
ν CO = 1720 et 1665 cm⁻¹
- solvt. de recristallisation :: isopropanol

### Exemple 9

### 2-[4-(2,5-Diméthoxyphényl)-semicarbazono]indane-1,3-dione

- Matières premières:: Ninhydrine Chlorhydrate de 4-(2,5-diméthoxyphényl)-semicarbazide
- Rdt. :: 75 %
- F :: 222°C
- IR :: ν NH = 3340 et 3200 cm⁻¹
ν CO = 1730 et 1690 cm⁻¹
- solvt. de recristallisation :: isopropanol

### Exemple 10

### 2-[4-(3,5-Diméthoxyphényl)-semicarbazono]indane-1,3-dione

- Matières premières :: Ninhydrine Chlorhydrate de 4-(3,5-diméthoxyphényl)-semicarbazide
- Rdt. :: 80 %
- F :: 234°C
- IR :: ν NH = 3380 et 3230 cm⁻¹
ν CO = 1730 et 1690 cm⁻¹
- solvt. de recristallisation :: acétate d'éthyle

### Exemple 11

### 2-[4-(3,4-Diméthoxyphényl)-semicarbazono]indane-1,3-dione

- Matières premières :: Ninhydrine Chlorhydrate de 4-(3,4-diméthoxyphényl)-semicarbazide
- Rdt. :: 70 %
- F :: 210°C
- IR :: ν NH = 3400 et 3260 cm⁻¹
ν CO = 1720 et 1670 cm⁻¹
- solvt. de recristallisation :: acétate d'éthyle

### Exemple 12

### 2-[4-(2-Chloro-5-méthoxyphényl)-semicarbazono]indane-1,3-dione

- Matières premières :: Ninhydrine Chlorhydrate de 4-(2-chloro-5-méthoxyphényl)-semicarbazide
- Rdt. :: 80 %
- F :: 228°C
- IR :: ν NH = 3330 et 3260 cm⁻¹
ν CO = 1730 et 1680 cm⁻¹
- solvt. de recristallisation :: acétate d'éthyle

### Exemple 13

### 2-(4,4-Diphénylsemicarbazono)-indane-1,3-dione

- Matières premières :: Ninhydrine Chlorhydrate de 4,4-diphénylsemicarbazide
- Rdt. :: 45 %
- F :: 256°C
- IR :: ν NH = 3200 cm⁻¹
ν CO = 1720,1700 et 1670 cm⁻¹
- solvt. de recristallisation :: méthanol

### Exemple 14

### 2-[4-(2-Méthylphényl)-semicarbazono]indane-1,3-dione

- Matières premières :: Ninhydrine Chlorhydrate de 4-(2-méthylphényl)-semicarbazide
- Rdt. :: 60 %
- F :: 192°C IR : ν NH = 3340 et 3210 cm⁻¹
ν CO = 1720 et 1675 cm⁻¹
- solvt. de recristallisation :: éthanol

### Exemple 15

### 2-[4-(3-Méthylphényl)-semicarbazono]indane-1,3-dione, monohydrate

- Matières premières :: Ninhydrine Chlorhydrate de 4-(3-méthylphényl)-semicarbazide
- Rdt. :: 95 %
- F :: 128°C
- IR :: ν NH = 3260 et 3240 cm⁻¹
ν CO = 1720 et 1670 cm⁻¹
- solvt. de recristallisation :: méthanol

### Exemple 16

### 2-[4-(4-Méthylphényl)-semicarbazono]indane-1,3-dione

- Matières premières :: Ninhydrine Chlorhydrate de 4-(4-méthylphényl)-semicarbazide
- Rdt. :: 74 %
- F :: 208°C
- IR :: ν NH = 3380 et 3200 cm⁻¹
ν CO = 1720 et 1670 cm⁻¹
- solvt. de recristallisation :: éther éthylique

### Exemple 17

### 2-[4-(4-Propylphényl)-semicarbazono]indane-1,3-dione

- Matières premières :: Ninhydrine Chlorhydrate de 4-(4-propylphényl)-semicarbazide
- Rdt. :: 74 %
- F :: 166°C
- IR :: ν NH = 3215 cm⁻¹
ν CO = 1710 et 1680 cm⁻¹
- solvt. de recristallisation :: éthanol

### Exemple 18

### 2-[4-(4-Tert-butylphényl)-semicarbazono]indane-1,3-dione

- Matières premières :: Ninhydrine Chlorhydrate de 4-(4-tert-butylphényl)-semicarbazide
- Rdt. :: 40 %
- F :: 258°C
- IR :: ν NH = 3360 et 3200 cm⁻¹
ν CO = 1710 et 1670 cm⁻¹
- solvt. de recristallisation :: éthanol

### Exemple 19

### 2-[4-(2-Chlorophényl)-semicarbazono]indane-1,3-dione

- Matières premières :: Ninhydrine Chlorhydrate de 4-(2-chlorophényl)-semicarbazide
- Rdt. :: 75 %
- F :: 208°C
- IR :: ν NH = 3330 et 3220 cm⁻¹
ν CO = 1710 et 1670 cm⁻¹
- solvt. de recristallisation :: acétate d'éthyle

### Exemple 20

### 2-[4-(2-Chloro-6-méthylphényl)-semicarbazono]indane-1,3-dione

- Matières premières :: Ninhydrine Chlorhydrate de 4-(2-chloro-6-méthylphényl)-semicarbazide
- Rdt. :: 75 %
- F :: 228°C
- IR :: ν NH = 3320 et 3200 cm⁻¹
ν CO = 1710 et 1670 cm⁻¹
- solvt. de recristallisation :: éthanol

### Exemple 21

### 2-[4-(4-Chlorophényl)-semicarbazono]indane-1,3-dione

- Matières premières :: Ninhydrine Chlorhydrate de 4-(4-chlorophényl)-semicarbazide
- Rdt. :: 75 %
- F :: 234°C
- IR :: ν NH = 3300 et 3280 cm⁻¹
ν CO = 1730 et 1680 cm⁻¹
- solvt. de recristallisation :: isopropanol

### Exemple 22

### 2-[4-(2,5-Diéthoxyphényl)-semicarbazono]indane-1,3-dione

- Matières premières :: Ninhydrine Chlorhydrate de 4-(2,5-diéthoxyphényl)-semicarbazide
- Rdt. :: 40 %
- F :: 226°C
- IR :: ν NH = 3340 cm⁻¹
ν CO = 1720 et 1675 cm⁻¹
- solvt. de recristallisation :: isopropanol

### Exemple 23

### 2-(4-Ethylthiosemicarbazono)-indane-1,3-dione

- Matières premières :: Ninhydrine Chlorhydrate de 4-éthylthiosemicarbazide
- Rdt. :: 30 %
- F :: 212°C
- IR :: ν NH = 3340 et 3240 cm⁻¹
ν CO = 1720 et 1670 cm⁻¹
- solvt. de recristallisation :: éthanol

### Exemple 24

### 2-(4-Méthylthiosemicarbazono)-indane-1,3-dione

- Matières premières :: Ninhydrine Chlorhydrate de 4-méthylthiosemicarbazide
- Rdt. :: 70 %
- F :: 234°C
- IR :: ν NH = 3200 cm⁻¹
ν CO = 1710 et 1670 cm⁻¹
- solvt. de recristallisation :: mélange (V/V) méthanol/acétate d'éthyle

### Exemple 25

### 2-[4-(4-Ethylphényl)-semicarbazono]indane-1,3-dione

- Matières premières :: Ninhydrine Chlorhydrate de 4-(4-éthylphényl)-semicarbazide
- Rdt. :: 60 %
- F :: 180°C
- IR :: ν NH = 3330 et 3220 cm⁻¹
ν CO = 1715 et 1670 cm⁻¹
- solvt. de recristallisation :: éthanol

### Exemple 26

### 2-[4-(3-Ethoxyphényl)-semicarbazono]indane-1,3-dione

- Matières premières :: Ninhydrine Chlorhydrate de 4-(3-éthoxyphényl)-semicarbazide
- Rdt. :: 85 %
- F :: 164°C
- IR :: ν NH = 3240 cm⁻¹
ν CO = 1730 et 1680 cm⁻¹
- solvt. de recristallisation :: méthanol

### Exemple 27

### 2-[4-(4-Ethoxyphényl)-semicarbazono]indane-1,3-dione

- Matières premières :: Ninhydrine Chlorhydrate de 4-(4-éthoxyphényl)-semicarbazide
- Rdt. :: 70 %
- F :: 200°C
- IR :: ν NH = 3300 et 3210 cm⁻¹
ν CO = 1720 et 1670 cm⁻¹
- solvt. de recristallisation :: acétate d'éthyle

### Exemple 28

### 2-[4-(2,4-Dichlorophényl)-semicarbazono]indane-1,3-dione

- Matières premières :: Ninhydrine Chlorhydrate de 4-(2,4-dichlorophényl)-semicarbazide
- Rdt. :: 35 %
- F :: 226°C
- IR :: ν NH = 3315 et 3210 cm⁻¹
ν CO = 1710 et 1670 cm⁻¹
- solvt. de recristallisation :: isopropanol

### Exemple 29

### 2-[4-(3,4-Méthylènedioxyphényl)-semicarbazono]indane-1,3-dione

- Matières premières :: Ninhydrine Chlorhydrate de 4-(3,4-méthylènedioxyphényl)-semicarbazide
- Rdt. :: 70 %
- F :: 228 °C
- IR :: ν NH = 3250 cm⁻¹
ν CO = 1715 et 1670 cm⁻¹
- solvt. de recristallisation :: acétate d'éthyle

### Exemple 30

### 2-[4-(2-Fluorophényl)-semicarbazono]indane-1,3-dione

- Matières premières :: Ninhydrine Chlorhydrate de 4-(2-fluorophényl)-semicarbazide
- Rdt. :: 80 %
- F :: 195°C
- IR :: ν NH = 3310 et 3210 cm⁻¹
ν CO = 1720 , 1700 et 1665 cm⁻¹
- solvt. de recristallisation :: méthanol/acétate d'ethyle (V/V)

### Exemple 31

### 2-[4-(3-Fluorophényl)-semicarbazono]indane-1,3-dione

- Matières premières :: Ninhydrine Chlorhydrate de 4-(3-fluorophényl)-semicarbazide
- Rdt. :: 90 %
- F :: 222°C
- IR :: ν NH = 3360 et 3230 cm⁻¹
ν CO = 1740 et 1695 cm⁻¹
- solvt. de recristallisation :: acétate d'éthyle

### Exemple 32

### 2-[4-(4-Fluorophényl)-semicarbazono]indane-1,3-dione

- Matières premières :: Ninhydrine Chlorhydrate de 4-(4-fluorophényl)-semicarbazide
- Rdt. :: 80 %
- F :: 230°C
- IR :: ν NH = 3320 et 3200 cm⁻¹
ν CO = 1710 et 1660 cm⁻¹
- solvt. de recristallisation :: acétate d'éthyle

### Exemple 33

### 2-[4-(2-Bromophényl)-semicarbazono]indane-1,3-dione

- Matières premières :: Ninhydrine Chlorhydrate de 4-(2-bromophényl)-semicarbazide
- Rdt. :: 80 %
- F :: 235°C
- IR :: ν NH = 3320 et 3220 cm⁻¹
ν CO = 1715 et 1680 cm⁻¹
- solvt. de recristallisation :: acétate d'éthyle

### Exemple 34

### 2-[4-(4-Bromophényl)-semicarbazono]indane-1,3-dione

- Matières premières :: Ninhydrine Chlorhydrate de 4-(4-bromophényl)-semicarbazide
- Rdt. :: 80 %
- F :: 236°C
- IR :: ν NH = 3230 cm⁻¹
ν CO = 1750 , 1730 et 1690 cm⁻¹
- solvt. de recristallisation :: acétate d'éthyle

### Exemple 35

### 2-[4-(2-Hydroxyéthyl)-semicarbazono]indane-1,3-dione

- Matières premières :: Ninhydrine Chlorhydrate de 4-(2-hydroxyéthyl)-semicarbazide
- Rdt. :: 70 %
- F :: 247°C
- IR :: ν OH/NH = 3360,3240 et 3220 cm⁻¹
ν CO = 1710 et 1680 cm⁻¹
- solvt. de recristallisation :: méthanol

### Exemple 36

### 2-[4-(2-Acétoxyphényl)-semicarbazono]indane-1,3-dione

A une solution de 1g(0,003 mole) de la 2-[4-(2-Hydroxyphényl)-semicarbazono]indane-1,3-dione (exemple 4) dans 10ml d'anhydride acétique, on ajoute 2 gouttes d'acide sulfurique concentré.

Le mélange réactionnel est agité à température ambiante pendant 10 minutes puis versé dans l'eau glacée.Le précipité jaune obtenu est essoré,lavé plusieurs fois à l'eau,séché et recristallisé dans l'acétonitrile.
- Rdt. :: 45 %
- F :: 242°C
- IR :: ν NH = 3320 et 3180 cm⁻¹
ν CO = 1740 , 1715 et 1675 cm⁻¹

### Exemple 37

### 5-Méthoxy-2-(4-phénylsemicarbazono)-indane-1,3-dione

- Matières premières :: 5-Méthoxyindane-1,2,3-trione, monohydrate Chlorhydrate de 4-phénylsemicarbazide
- Rdt.: : 61 %
- F :: 230°C
- IR :: ν NH = 3250 cm⁻¹
ν CO = 1720 et 1680 cm⁻¹
- solvt. de recristallisation :: éthanol

### Exemple 38

### 5-Méthoxy-2-(4-phénylthiosemicarbazono)-indane-1,3-dione

- Matières premières :: 5-Méthoxyindane-1,2,3-trione, monohydrate Chlorhydrate de 4-phénylthiosemicarbazide
- Rdt. :: 48 %
- F :: 149°C
- IR :: ν NH = 3260 cm⁻¹
ν CO = 1720 et 1680 cm⁻¹
- solvt. de recristallisation :: éthanol

### Exemple 39

### 5-Méthoxy-2-semicarbazono-indane-1,3-dione

- Matières premières :: 5-Méthoxyindane-1,2,3-trione, monohydrate Chlorhydrate de semicarbazide
- Rdt. :: 86 %
- F :: > 265°C
- IR :: ν NH = 3320 et 3220 cm⁻¹
ν CO = 1720 et 1680 cm⁻¹
- solvt. de recristallisation :: mélange (V/V) éthanol/acétate d'éthyle

### Exemple 40

### 5-Méthoxy-2-[4-(2-hydroxyphényl)-semicarbazono]indane-1,3-dione

- Matières premières :: 5-Méthoxyindane-1,2,3-trione, monohydrate Chlorhydrate de 4-(2-hydroxyphényl)-semicarbazide reflux 5 heures
- Rdt. :: 60 %
- F :: 262°C
- IR :: ν OH = 3400 cm⁻¹
ν NH = 3300 cm⁻¹
ν CO = 1750,1700 et 1620 cm⁻¹
précipité lavé à l'ether

### Exemple 41

### 5-Méthoxy-2-thiosemicarbazono-indane-1,3-dione

- Matières premières :: 5-Méthoxyindane-1,2,3-trione, monohydrate Chlorhydrate de thiosemicarbazide
- Rdt. :: 43 %
- F :: > 265°C
- IR :: ν NH = 3220 et 3120 cm⁻¹
ν CO = 1710 et 1655 cm⁻¹
- solvt. de recristallisation :: éthanol

### Exemple 42

### 5-hydroxy-2-(4-phénylsemicarbazono)-indane-1,3-dione

- Matières premières :: 5-Hydroxyindane-1,2,3-trione,monohydrate Chlorhydrate de 4-phénylsemicarbazide reflux 4 heures
- Rdt :: 70 %
- F :: 265°C
- IR :: ν OH = 3400 cm⁻¹
ν NH = 3240 et 3220 cm⁻¹
ν CO = 1680 cm⁻¹
- solvt. de recristallisation :: acétonitrile

### Exemple 43

### 5-hydroxy-2-semicarbazono-indane-1,3-dione

- Matières premières :: 5-Hydroxyindane-1,2,3-trione,monohydrate Chlorhydrate de semicarbazide reflux 4 heures
- Rdt :: 50 %
- F :: > 265°C
- IR :: ν OH/NH₂ = 3420 cm⁻¹
ν CO = 1720 et 1660 cm⁻¹
- solvt. de recristallisation :: éthanol

### Exemple 44

### 5-Hydroxy-2-(4-phénylthiosemicarbazono)-indane-1,3-dione

- Matières premières :: 5-Hydroxyindane-1,2,3-trione,monohydrate Chlorhydrate de 4-phénylthiosemicarbazide reflux 2 heures
- Rdt. :: 45 %
- F :: > 265°C
- IR :: ν OH = 3400 cm⁻¹
ν NH = 3300 et 3120 cm⁻¹
ν CO = 1710 et 1675 cm⁻¹
- solvt. de recristallisation :: mélange (V/V) acétate d'éthyle/éther de pétrole

### Exemple 45

### 5-Hydroxy-2-thiosemicarbazono-indane-1,3-dione

- Matières premières :: 5-Hydroxyindane-1,2,3-trione,monohydrate Chlorhydrate de thiosemicarbazide reflux 2 heures
- Rdt. :: 78 %
- F :: > 265°C
- IR :: ν OH = 3420 cm⁻¹
ν NH = 3300 et 3120 cm⁻¹
ν CO = 1710 et 1680 cm⁻¹
- solvt. de recristallisation :: mélange (V/V) acétate d'éthyle/éther de pétrole

### Exemple 46

### 5,6-Diméthoxy-2-semicarbazono-indane-1,3-dione,monohydrate

- Matières premières :: 5,6-Diméthoxyindane-1,2,3-trione,monohydrate Chlorhydrate de semicarbazide
- Rdt. :: 60 %
- F :: > 265°C
- IR :: ν NH = 3420 et 3300 cm⁻¹
ν CO = 1730 et 1680 cm⁻¹
- solvt. de recristallisation :: méthanol

### Exemple 47

### 5,6-Diméthoxy-2-(4-phénylsemicarbazono)-indane-1,3-dione,monohydrate

- Matières premières :: 5,6-Diméthoxyindane-1,2,3-trione,monohydrate Chlorhydrate de 4-phénylsemicarbazide
- Rdt. :: 80 %
- F :: > 265°C
- IR :: ν NH = 3240 cm⁻¹
ν CO = 1730,1710 et 1670 cm⁻¹
- solvt. de recristallisation :: méthanol

### Exemple 48

### 2-[4-(3-Trifluorométhylphényl)-semicarbazono]indane-1,3-dione

- Matières premières :: Ninhydrine Chlorhydrate de 4-(3-trifluorométhylphényl)-semicarbazide
- Rdt. :: 70 %
- F :: 230° C
- IR :: ν NH = 3360 et 3180 cm⁻¹
ν CO = 1730 et 1680 cm⁻¹
- solvt. de recristallisation :: acétate d'éthyle

### Exemple 49

### 2-[4-(2-Trifluorométhylphényl)-semicarbazono]indane-1,3-dione

- Matières premières :: Ninhydrine Chlorhydrate de 4-(2-trifluorométhylphényl)-semicarbazide
- Rdt. :: 70 %
- F. :: 160° C
- IR :: ν NH = 3360 et 3180 cm⁻¹
ν CO = 1740 et 1680 cm⁻¹
- solvt. de recristallisation :: mélange (V/V) éther/isopropanol

### Exemple 50

### 2-[4-(3-Bromophényl)-semicarbazono]indane-1,3-dione

- Matières premières :: Ninhydrine Chlorhydrate de 4-(3-trifluorométhylphényl)-semicarbazide
- Rdt. :: 75 %
- F. :: 180° C
- IR :: ν NH = 3360 et 3180 cm⁻¹
ν CO = 1730 et 1680 cm⁻¹
- solvt. de recristallisation :: méthanol

### Exemple 51

### 2-[4-(4-Bromophényl)-thiosemicarbazono]indane-1,3-dione

- Matières premières :: Ninhydrine Chlorhydrate de 4-(4-bromophényl)-thiosemicarbazide
- Rdt. :: 50 %
- F. :: 230° C
- IR :: ν NH = 3340 cm⁻¹
ν CO = 1730 et 1700 cm⁻¹

### Exemple 52

### 2-[4-(pyridin-4-yl) semicarbazono]indane-1,3-dione

A 70 mmoles de 2,2-dihydroxy-1,3-dioxo-2H-indène en solution dans 100 ml d'éthanol, on ajoute à température ambiante 70 mmoles de 4-pyridylsemicarbazide en suspension dans 100 ml d'éthanol. Le mélange réactionnel qui prend une coloration jaune est maintenu sous agitation à température ambiante pendant 24 h. Le précipité obtenu est filtré sur verre fritté, lavé à l'éther et au pentane. Les cristaux jaunes sont purifiés sur colonne de silice moyenne pression et recristallisés. Ils sont séchés une nuit sous vide à 50° C.
- -Rdt :: 74%
- -F :: >260°C
- -IR (KBr): ν NH : 3300 cm⁻¹,
ν C=O : 1730 et 1670 cm⁻¹
- -RMN du ¹H (200 MHz, DMSO d₆) :: δ (ppm)
10.65 (1s, 1H, NH)
8.45 (d, 2H, H-2', H6', J2'-3' et 5'-6'=6Hz)
8.00 (s,4H, H-4, H-5, H-6, H-7)
7.65 (d, 2H, H-3', H-5', J2'-3' et 5'-6'=6Hz)
- solvant de recristallisation:: acétate d'éthyle

### Exemple 53

### 2-[4-(2,4-difluorophényl)semicarbazono]indane-1,3-dione

- Matières premières :: 2,2-dihydroxy-1,3-dioxo-2H-indène 4-(2,4-difluorophényl)semicarbazide, chlorhydrate
- -Rdt :: 80%
- -F :: 231°C
- -IR (KBr): ν NH : 3300 et 3220 cm⁻¹,
ν C=O : 1725 et 1700 cm⁻¹
- -RMN du ¹H (200 MHz, DMSO d₆) :: δ (ppm)
13.53 (1s, 1H, NH)
12.21 (1s, 1H, NH)
7.98 (s ,4H, H-4, H-5,H-6, H-7)
7.93 (m, 1H, H-3')
7.30 (m, 1H, H-5')
7.10 (s, 1H, H-6')
- solvant de recristallisation:: acétate d'éthyle

### Exemple 54

### 2-[4-(2,6-difluorophényl)semicarbazono]indane-1,3-dione

- Matières premières :: 2,2-dihydroxy-1,3-dioxo-2H-indène 4-(2,6-difluorophényl)semicarbazide, chlorhydrate
- -Rdt :: 74%
- -F :: 268°C
- -IR (KBr): ν NH : 3300 et 3220 cm⁻¹,
ν C=O : 1725 et 1700 cm⁻¹
- -RMN du ¹H (200 MHz, DMSO d₆) :: δ (ppm)
12,47 (1s, 1H, NH)
9,98 (1s, 1H, NH)
8,00 (s ,4H, H-4, H-5,H-6, H-7)
7,49 (m, 1H, H-4')
7,27 (t, 2H, H-3',H-5', JH5'-4' = JH3'-4' = 7.91Hz))
- solvant de recristallisation:: acétate d'éthyle

### Exemple 55

### 2-[4-(4-trifluorométhylphényl)semicarbazono]indane-1,3-dione

- Matières premières :: 2,2-dihydroxy-1,3-dioxo-2H-indène 4-(4-trifluorométhylphényl)semicarbazide, chlorhydrate
- -Rdt :: 90%
- -F :: 254°C (décomposition)
- -IR (KBr): ν NH : 3280 et 3220 cm⁻¹,
ν C=O : 1740 et 1700 cm⁻¹
- -RMN du ¹H (200 MHz, DMSO d₆) :: δ (ppm)
12,56 (1s, 1H, NH)
10,73 (1s, 1H, NH)
8,00 (s ,4H, H-4, H-5,H-6, H-7)
7.80 (d, 2H, H-3',H-5', JH3'-2'et 6'-5' = 8.4 Hz)
7.72 (d, 2H, H-2',H-6',JH3'-2'et 6'-5' = 8.4 Hz)
- solvant de recristallisation:: dichlorométhane

### Exemple 56

### 2-[4-(2,4-difluoro-6-éthoxyphényl)semicarbazono]indane-1,3-dione

- Matières premières :: 2,2-dihydroxy-1,3-dioxo-2H-indène 4-(2,4-difluoro-6-éthoxyphényl)semicarbazide, chlorhydrate
- -Rdt :: 79%
- -F :: 203°C
- -IR (KBr): ν NH: 3240cm⁻¹,
ν C=O : 1730;17100 et 1670 cm⁻¹
- -RMN du ¹H (200 MHz, DMSO d₆) :: δ (ppm)
13,13 (s, 1H, NH)
9,28 (s, 1H, NH)
7,94 (s, 4H, H-4, H-5,H-6, H-7)
7,31 (m, 1H, H-3')
7,19 (m, 1H, H-5')
4,20 (q, 2H, CH_{2,} J = 8Hz)
1,32 (t, 3H, CH_{3,} J = 8Hz)
- solvant de recristallisation:: dichlorométhane

### Exemple 56

### 2-[4-(4-fluorophényl)thiosemicarbazono]indane-1,3-dione

- Matières premières :: 2,2-dihydroxy-1,3-dioxo-2H-indène 4-(4-fluorophényl)thiosemicarbazide, chlorhydrate
- -Rdt :: 60%
- -F :: 209°C
- -IR (KBr): ν NH : 3270 et 3210 cm⁻¹,
ν C=O : 1725 et 1680 cm⁻¹
- -RMN du ¹H (200 MHz, DMSO d₆) :: δ (ppm)
12,85 (1s, 1H, NH)
11,24 (1s, 1H, NH)
8,00 (s ,4H, H-4, H-5,H-6, H-7)
7,60 (m, 2H, H-3',H-5')
7,24 (t, 2H, H-2',H-6', JH2'-3'et 5'-6' = 8.9 Hz)
- solvant de recristallisation:: dichlorométhane

### Exemple 58

### 2-[4-(2-fluorophényl)thiosemicarbazono]indane-1,3-dione

- Matières premières :: 2,2-dihydroxy-1,3-dioxo-2H-indène (2-fluorophényl)thiosemicarbazide, chlorhydrate
- -Rdt :: 77%
- -F :: 224°C
- -IR (KBr): ν NH : 3240 et 3200 cm⁻¹,
ν C=O : 1720 et 1680 cm⁻¹
- -RMN du ¹H (200 MHz, DMSO d₆) :: δ (ppm)
12,89 (1s, 1H, NH)
11,00 (1s, 1H, NH)
8,00 (s ,4H, H-4, H-5,H-6, H-7)
7,49 (m, 1H, H-3')
7,34 (m, 3H, H-4',H-5',H-6')
- solvant de recristallisation:: éther éthylique

### E/ Préparation des composés de formule (If)

### Méthode ⑤

A une solution de 5,6mmoles de 2,2-Dihydroxy-1,3-dioxo-2H-indène dans 30ml d'éthanol, on ajoute 40ml d'une solution aqueuse contenant 5,6mmoles d' hydrazinocarbonylhydrazine d'hydrazinothiocarbonylhydrazine.

Le mélange réactionnel est agité à température ambiante pendant sept heures et le précipité blanc obtenu est essoré, lavé à l'éthanol et séché. Ce précipité est ensuite mis en suspension dans 20ml d'ethanol, puis on ajoute deux gouttes d'acide chlorhydrique concentré. Le mélange est porté au reflux pendant trente minutes et le produit jaune-orangé formé est essoré, séché, puis recristallisé.

### Exemple 59

### 2-(phénylhydrazinocarbonylhydrazono)indane-1,3-dione

- Matières premières :: 2,2-dihydroxy-1,3-dioxo-2H-indène phénylhydrazinocarbonylhydrazine
- -Rdt :: 98%
- -F :: 245°C
- -IR (KBr): ν NH : 3320 cm⁻¹,
ν C=O : 1740,1720 et 1680 cm⁻¹
- -RMN du ¹H (200 MHz, DMSO d₆) :: δ (ppm)
13.25 (s, 1H, NH)
9,37 (s, 1H, NH)
8,17 (s, 1H, NH)
7.95 (s ,4H, H-4, H-5,H-6, H-7)
7.23 (m, 2H, H-2',H-6')
7.30 (m, 3H, H-3',H-4',H-5')
- solvant de recristallisation:: acétone

### Exemple 60

### 2-[(4-fluorophényl)hydrazinocarbonylhydrazono]indane-1,4-dione

- Matières premières :: 2,2-dihydroxy-1,3-dioxo-2H-indène (4-Fluorophényl)hydrazinocarbonylhydrazine
- -Rdt :: 67%
- -F :: 250°C
- -IR (KBr): ν NH : 3240 et 3320 cm⁻¹,
ν C=O : 1740 et 1700cm⁻¹
- -RMN du ¹H (200 MHz, DMSO d₆) :: δ (ppm)
13.25 (1s, 1H, NH)
9,43 (1s, 1H, NH)
8,20 (1s, 1H, NH)
7.95 (s ,4H, H-4, H-5,H-6, H-7)
7.10 (m, 2H, H-3',H-5')
6,88 (m, 2H, H-2',H-6')
- solvant de recristallisation:: éthanol/acétone 50/50%

### F/ Préparation des composés de formule (Ig)

### Méthode ⑥

A une solution de 30 mmoles de 2,2-dihydroxy-1,3-dioxo-2H-indène dans 70 ml d'éthanol, on ajoute 10 ml d'une solution aqueuse contenant 30 mmoles d'(Arylimino)méthylthiohydrazine.

Le mélange réactionnel est porté à 60°C pendant trente minutes et le précipité marron-orangé obtenu est essoré à chaud,séché puis recristallisé.

### Exemple 61

### 2-[(phénylimino)méthylthiohydrazono]indane-1,3-dione

- Matières premières :: 2,2-dihydroxy-1,3-dioxo-2H-indène (Phénylimino)méthylthiohydrazine
- Rdt :: 40%
- -F :: 200°C
- -IR (KBr): ν NH : 3200 cm⁻¹,
ν C=O : 1720 et 1690 cm⁻¹
- -RMN du ¹H (200 MHz, DMSO d₆) :: δ (ppm)
12,75 (1s, 1/2H, NH)
9,63 (1s, 1/2H, NH)
7,98 (d,1H, H-2' ou H-6',JH2'-3' = 7.4 Hz)
7.92 (s ,4H, H-4, H-5,H-6, H-7)
7.44 (m, 2H, H-3',H-5')
7.21 (t, 1H, H-4', JH4'-5' = 7.4)
6,97 (d, 1H, H-2' ou H-6', JH2'-3' = 7.4 Hz)
2,50 (s, 3H, CH₃)
- solvant de recristallisation:: acétate d'éthyle

### Exemple 62

### 2-[(4-fluorophénylimino)méthylthiodydrazono]indane-1,3-dione

- Matières premières :: 2,2-dihydroxy-1,3-dioxo-2H-indène (4-Fluorophénylimino)méthylthiohydrazine
- -Rdt :: 50%
- -F :: >260°C
- -IR (KBr): ν NH : 3180 cm⁻¹,
ν C=O : 1740 et 1680 cm⁻¹
- -RMN du ¹H (200 MHz, DMSO d₆) :: δ (ppm)
12,86 (s, 1/2H, NH)
9,78 (s, 1/2H, NH)
8,14 (d,1H, H-2' ou H-6',JH2'-3' = 7.4 Hz)
8,00 (s ,4H, H-4, H-5,H-6, H-7)
7.37 (m, 2H, H-3',H-5')
7,13 (d, 1H, H-2' ou H-6',JH2'-3' = 7.4 Hz)
2,61 (s, 3H, CH₃)
- solvant de recristallisation:: acétone

### G/ Préparation des composes de formules (Ii)-(Ik)

### Méthode ⑦

### Exemple 63

### 1,2-(4-Phénylsemicarbazono)-3-indanone

- Matières premières :: Ninhydrine Chlorhydrate de 4-phénylsemicarbazide
- Rdt. :: 70 %
- F :: > 260°C
- IR :: ν NH = 3360,3325 et 3300 cm⁻¹
ν CO = 1700 et 1675 cm⁻¹
précipité lavé à l'éthanol

### Exemple 64

### 1-Oximino-2-(4-phénylsemicarbazono)-3-indanone

A une solution de 0,01 mole de 2-(4-phénylsemicarbazono)-indane-1,3-dione dans 150 ml d'éthanol,on ajoute 0,01 mole de chlorhydrate d'hydroxlamine et de 0,01 mole d'acétate de sodium en solution dans 20 ml d'eau.Le mélange réactionnel est porté au reflux pendant 1 heure,puis l'ethanol est éliminé sous pression réduite.Les cristaux formés sont essorés,laves à l'eau,séchés et recristallisés dans l'isopropanol.
- Rdt. :: 70 %
- F. :: 240° C
- IR :: ν OH = 3400 cm⁻¹
ν NH = 3300 et 3240 cm⁻¹
ν CO = 1710 cm⁻¹

### H/ Préparation des composés de formules (Il) et (Im)

### Méthode ⑨

### Exemple 65

### 1,3-Dioximino-2-(4-phénylsemicarbazono)-indane

A une solution de 0,01 mole de 2-(4-phénylsemicarbazono)-indane-1,3-dione dans 150 ml d'éthanol,on ajoute 0,02 mole de chlorhydrate d'hydroxylamine et de 0,01 mole d'acétate de sodium en solution dans 20 ml d'eau.Le mélange réactionnel est porté au reflux pendant 1 heure,puis l'éthanol est éliminée sous pression réduite.Les cristaux formés sont essorés,lavés à l'eau,séchés et recristallisés dans l'acétate d'éthyle.
- Rdt. :: 65 %
- F :: > 265°C
- IR :: ν OH = 3450 cm⁻¹
ν NH = 3180 cm⁻¹
ν CO = 1680 cm⁻¹
Il convient de préciser que les spectres infrarouges ci-dessus ont été déterminés avec des pastilles KBr.

Par ailleurs, on donne ci-après les spectres RMN des composés objet des exemples ci-dessus. Ces spectres ont été déterminés sur un appareil de RMN, 200 MHz, en solution dans le DMSO (D₆). Ils sont décrits selon le protocole suivant : déplacement (δ) en ppm, forme du signal, nombre de protons, nature des protons, constantes de couplage s'il y a lieu.

La numérotation des protons aromatiques sur l'indane est comme suit : 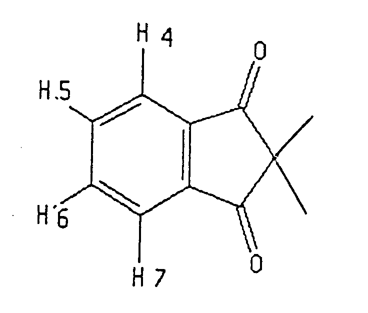
Dans le cas où R et R₁ comportent un hétérocycle ou forment un groupe comportant un hétérocycle, la numérotation des protons de cet hétérocycle se fera à partir de l'hétéroatome en direction de la liaison sur le substrat principal.

### Spectre RMN

### -exemple a, 5-Hydroxyindane-1,3-dione

7.78 (d, 1H, H-7, J H6-7 = 8.3 Hz)
7.26 (d ,1H, H-6, J H6-7 = 8.3 Hz)
7.11 (1s, 1H, H-4)
3.25 (s, 3H, CH2)

### -exemple b, 2-Bromo-5-hydroxyindane-1,3-dione

11,55 (1s, OH)
7.90 (dd, 1H, H-6, J H6-4 =2.93 Hz ; J H6-7 = 8.3 Hz)
7.39 (d, 1H, H-7, J H6-7 = 8.3 Hz)
7.21 (s, 1H, H-4)
5.50 (s, 1H, H2)

### -exemple c, 5-Hydroxyindane-1,2,3-trione, monohydrate

11.50 (1s, 1H, OH)
7.90 (d, 1H, H7, J H6-7 = 5.9 Hz)
7.40 (m, 2H, H4, H6)

### -exemple d, 5,6-Dimethoxyindane-1,2,3-trione, monohydrate

7.40 (1s, 2H, H-4, H-7)
4.03 (s, 6H, 2CH3O)

### -exemple e, 6-Hydroxy-5-methoxyindane-1,2,3-trione, monohydrate

11.21 (1s, 1H, OH)
7.28 (m, 2H, H-4, H-7)
4.03 (s, 6H, 2CH3O)

### exemple f, 2-oximino-5-hydroxyindane-1,3-dione

9.16 (1s, 1H, OH)
7.83 (dd, 1H, H6, JH6-7 = 9.0 Hz et JH4-6 = 3.0 Hz)
7.21 (m, 2H, H7, H4)

### -exemple 1, 2-Hydroxy-2-[4-2-hydroxyphényl)-semicarbazido]indane-1,3-dione

9.55 (1s, 1H, NH)
8.27 (s, 1H, OH)
8.00 (s, 4H, H-4, H-5, H-6, H-7)
7.90 (m, 2H, NH, H-3')
7.13 (1s, 1H, OH)
6.71 (m, 3H, H-4', H-5', H-6')
6.30 (1s, 1H, NH)

### exemple 2, 2-semicarbazono-indane-1,3-dione

12.55 (s, 1H,NH)
7.95 (s, 4H H-4, H-5, H-6, H-7)
7.41 (s, 2H, NH2)

### -exemple 3, 2-(4-Phénylsemicarbazono)-indane-1,3-dione

12.55 (s, 1H, NH)
10.35 (s, 1H, NH)
8.00 (s, 4H, H-4, H-5, H-6, H-7)
7.65 (d, 2H, H-2', H-6', J H2'-3' et 5'-6' = 8.2 Hz)
7.35 (dd, 2H, H-3', H-5', J H2'-3' et 5'-6' = 8.2 Hz)
7.10 (m, 1H, H-4')

### -exemple 4, 2-[4-(2-hydroxyphényl)-semicarbazono]indane-1,3-dione

12.55 (s, 1H, NH)
10.18 (s, 1H, OH)
9.64 (s, 1H, NH)
7.97 (s, 5H, H-4, H-5, H-6, H-7, H-6')
6.92 (m, 3H, H-3', H-4', H-5')

### -exemple 5, 2-(4-Phénylthiosemicarbazono)indane-1,3-dione

12.50 (1s, 1H, NH)
10.35 (1s, 1H, NH)
7.95 (s, 4H, H-4, H-5, H-6, H-7)
7.58 (d, 2H, H-2', H-6', J H2'-3' et 5'-6' = 8.3 Hz)
7.34 (dd, 2H, H-3', H-5', J H2'-3' et 5'-6' = 8.3 Hz)
7.07 (dd, 1H, H-4', J H4'-5' et 3'-4' = 8.3 Hz)

### -exemple 6, 2-[4-(2-Méthoxyphényl)-semicarbazono]indane-1,3-dione

12.57 (s, 1H, NH)
9.69 (s, 1H, NH)
8.03 (m, 1H, H-6')
7.95 (s, 4H, H-4, H-5, H-6, H-7)
7.06 (m, 2H, H-3', H-5')
6.97 (m, 1H, H-4')
3.89 (s, 3H, CH3)

### -exemple 7, 2-[4-(3-Méthoxyphényl)-semicarbazono]indane-1,3-dione

12.48 (1s, 1H, NH)
10.32 (1s, 1H, NH)
7.96 (s, 4H, H-4, H-5, H-6, H-7)
7.22 (m, 3H, H-4', H-5', H-6')
6.65 (d, 1H, H-3', J H3'-4' = 7.8 Hz)
3.75 (s, 3H, CH3O)

### -exemple 8, 2-[4-(4-Méthoxyphényl)-semicarbanzono]indane-1,3-dione, monohydrate

12.46 (1s, 1H, NH)
10.22 (1s, 1H, NH)
7.96 (s, 4H, H-4, H-5, H-6, H-7)
7.50 (d, 2H, H-2', H-6', J H2'-3' et 5'-6' = 8.8 Hz)
6.92 (d, 2H, H-3', H-5', J H2'-3' et 5'-6' = 8.8 Hz)
3.73 (s, 3H, CH3O)

### -exemple 9, 2-[4-(2,5-Diméthoxyphényl)-semicarbazono]indane-1,3-dione

12.54 (1s, 1H, NH)
9.68 (1s, 1H, NH)
7.96 (s, 4H, H-4, H-5, H-6, H-7)
7.73 (m, 1H, H-6')
6.98 (d, 1H, H-3', J H3'-4' = 8.3 Hz)
6.63 (dd, 1H, H-4', J H3'-4' = 8.3 Hz)
3.83 (s, 3H, CH3O)
3.70 (s, 3H, CH3O)

### -exemple 10, 2-[4-(3,5-Diméthoxyphényl)-semicarbazono]indane-1,3-dione

12.50 (s, 1H, NH)
9.75 (s, 1H, NH)
8.00 (s, 4H, H-4, H-5, H-6, H-7)
6.85 (m, 2H, H-2', H-6')
6.63 (m, 1H, H-4')
3.75 (s, 6H, 2CH3O)

### -exemple 11, 2-[4-(3,4-Diméthoyphényl)-semicarbazono]indane-1,3-dione

12.50 (s, 1H, NH)
10.19 (s, 1H, NH)
7.97 (s, 4H, H-4, H-5, H-6, H-7)
7.20 (m, 2H, H-2', H-6')
6.92 (d, 1H, H-5', J H5'-6' = 8.8 MHz)
3.76 (s, 3H, CH3O)
3.73 (s, 3H, CH3O)

### -exemple 12, 2-[4-(2-Chloro-5-méthoxyphényl)-semicarbazono]indane-1,3-dione

12.63 (s, 1H, NH)
9.86 (s, 1H, NH)
7.93 (s, 4H, H-4, H-5, H-6, H-7)
7.62 (d, 1H, H-6', J H4'-6' = 3.0 Hz)
7.40 (d, 1H, H-3', J H3'-4' = 8.8 Hz)
6.76 (dd, 1H, H-4', J H3'-4' = 8.8 Hz et J H4'-6' = 3.0 Hz)
3.76 (s, 3H, CH3O)

### -exemple 13, 2-(4,4-Diphénylsemicarbazono)indane-1,3-dione

12.48 (s, 1H, NH)
7.93 (m, 5H, Ph)
7.89 (s, 4H, H-4, H-5, H-6, H-7)
7.85 (m, 5H, Ph)

### -exemple 14, 2-[4-(2-Méthylphényl)-semicarbazono]indane-1,3-dione

12.57 (s, 1H, NH)
9.63 (s, 1H, NH)
7.97 (s, 4H, H-4, H-5, H-6, H-7)
7.60 (d, 1H, H-6', J H 5'-6' = 7.7 Hz)
7.23 (m, 2H, H-3', H-5')
7.14 (m, 1H, H-4')
2.27 (s, 3H, CH3)

### -exemple 15, 2-[4-(3-Méthylphényl)-semicarbazono]indane-1,3-dione

12.51 (s, 1H, NH)
10.29 (s, 1H, NH)
7.97 (s, 4H, H-4, H-5, H-6, H-7)
7.38 (m, 2H, H-2', H-6')
7.24 (t, 1H, H-5', J H4'-5' et J H5'-6' = 7.3 Hz)
6.90 (d, 1H, H-4',J H4'-5' = 7.3 Hz)
2.30 (s, 3H, CH3)

### -exemple 16, 2-[4-(4-Méthylphényl)-semicarbazono]indane-1,3-dione

12.47 (s, 1H, NH)
10.26 (s, 1H, NH)
7.96 (s, 4H, H-4, H-5, H-6, H-7)
7.48 (d, 2H, H-2', H-6', J H2'-3' et J H5'-6' = 8.3 Hz)
7.14 (d, 2H, H-3', H-5', J H2'-3' et J H5'-6' = 8.3 Hz)
2.26 (s, 3H, CH3)

### -exemple 17, 2-[4-(4-Propylphényl)-semicarbazono]indane-1,3-dione

12.50 (s, 1H, NH)
10.29 (s, 1H, NH)
7.97 (s, 4H, H-4, H-5, H-6, H-7)
7.48 (d, 2H, H-2', H-6', J H2'-3' et J H5'-6' = 8.3 Hz)
7.16 (d, 2H, H-3', H-5', J H2'-3' et J H5'-6' = 8.3 Hz)
2.50 (m, 2H, CH2)
1.57 (m, 2H, CH2)
0.89 (t, 3H, CH3, J CH3-CH2= 6.8 Hz)

### -exemple 18, 2-[4-(4-Tert-butylphényl)-semicarbazono]indane-1,3-dione

12.50 (s, 1H, NH)
10.29 (s, 1H, NH)
7.97 (s, 4H, H-4, H-5, H-6, H-7)
7.49 (d, 2H, H-2', H-6', J H2'-3' et J H5'-6' = 8.3 Hz)
7.36 (d, 2H, H-3', H-5', J H2'-3' et J H5'-6' = 8.3 Hz)
1.27 (s, 9H, t-Bu)

### -exemple 19, 2-[4-(2-Chlorophényl)-semicarbazono]indane-1,3-dione

12.61 (s, 1H, NH)
9.91 (s, 1H, NH)
7.98 à 7.91 (1s, 5H, H-4, H-5, H-6, H-7, H-6')
7.53 (d, 1H, H-3', J H3'-4' = 7.3 Hz)
7.38 (t, 1H, H-5', J H5'-4' et J H5'-6' = 7.3 Hz)
7.20 (t, 1H, H-4', J H3'-5' et J H4'-5' = 7.3 Hz

### -exemple 20, 2-[4-(2-Chloro-6-méthylphényl)-semicarbazono]indane-1,3-dione

12.45 (s, 1H, NH)
9.82 (s, 1H, NH)
7.98 (s, 4H, H-4, H-5, H-6, H-7)
7.37-7.24 (m, 3H, H-3', H-4', H-5')
2.49 (s, 3H, CH3)

### -exemple 21, 2-[4-(4-Chlorophényl)-semicarbazono]indane-1,3-dione

12.50 (s, 1H, NH)
10.47 (s, 1H, NH)
7.97 (s, 4H, H-4, H-5, H-6, H-7)
7.60 (d, 2H, H-2', H-6', J H2'-3' et J H5'-6' = 8.8 Hz)
7.40 (d, 2H, H-3', H-5', J H2'-3' et J H5'-6' = 8.8 Hz)

### -exemple 22, 2-[4-(2,5-Diéthoxyphényl)-semicarbazono]indane-1,3-dione

12.50 (s, 1H, NH)
9.52 (s, 1H, NH)
7.99 (s, 4H, H-4, H-5, H-6, H-7)
7.71 (d, 1H, H-6', J H4'-6' = 2.9 Hz)
7.00 (d, 1H, H-3', J H3'-4' = 8.8 Hz)
6.63 (dd, 1H, H-4', J H3'-4' = 8.8 Hz et J H4'-6' = 2.9 Hz)
4.06 (q, 2H, CH2)
3.90 (q, 2H,CH2)
1.38 (m, 6H, 2CH3)

### -exemple 23, 2-(4-Ethylthiosemicarbazono)-indane-1,3-dione

12.57 (s, 1H, NH)
9.68 (s, 1H, NH)
7.97 (s, 4H, H-4, H-5, H-6, H-7)
3.63 (q, 2H, CH2, J CH3-CH2 = 6.35)
1.17 (d, 3H, CH3, J CH3-CH2 = 6.35)

### -exemple 24, 2-(4-Méthylthiosemicarbazono)-indane-1,3-dione

12.62 (s, 1H, NH)
10,00 (s, 1H, NH)
7.98 (s, 4H, H-4, H-5, H-6, H-7)
3.07 (s, 3H, CH3)

### -exemple 25, 2-[4-(4-Ethylphényl)-semicarbazono]-indane-1,3-dione

12.50 (s, 1H, NH)
10,27 (s, 1H, NH)
7.97 (s, 4H, H-4, H-5, H-6, H-7)
7.48 (d, 2H, H-2', H-6')
7.17 (d, 2H, H-3', H-5')
3.33 (q, 2H, CH2)
1.17 (m, 3H, CH3)

### -exemple 26, 2-[4-(3-Ethoxyphényl)-semicarbazono]-indane-1,3-dione

12.57 (s, 1H, NH)
10,30 (s, 1H, NH)
7.95 (s, 4H, H-4, H-5, H-6, H-7)
7.30 (m, 3H, H-2', H-5', H-6')
7.05 (d, 1H, H-4', J H4'-5' = 7.8 Hz)
3.93 (q, 2H, CH2, J CH3-CH2 = 6.35)
1.35 (t, 3H, CH3, J CH3-CH2 = 6.35)

### -exemple 27, 2-[4-(4-Ethoxyphényl)-semicarbazono]-indane-1,3-dione

12.47 (1s, 1H, NH)
10,17 (1s, 1H, NH)
7.97 (s, 4H, H-4, H-5, H-6, H-7)
7.45 (d, 2H, H-2', H-6', J H2'-3' = 8.8 Hz et J H5'-6' = 8.8 Hz)
6.90 (d, 2H, H-3', H-5', J H2'-3' = 8.8 Hz et J H5'-6' = 8.8 Hz)
3.98 (q, 2H, CH2, J CH3-CH2 = 6.8)
1.34 (t, 3H, CH3, J CH3-CH2 = 6.8)

### -exemple 28, 2-[4-(2,4-Dichlorophényl)-semicarbazono]-indane-1,3-dione

12.66 (s, 1H, NH)
10,01 (s, 1H, NH)
8.04 (s, 4H, H-4, H-5, H-6, H-7)
7.97 (d, 1H, H-6')
7.85 (s, 1H, H-3')
7.47 (d, 1H, H-5')

### -exemple 29, 2-[4-(3,4-Méthylènedioxyphényl)-semicarbazono]-indane-1,3-dione

12.44 (s, 1H, NH)
10,23 (s, 1H, NH)
7.96 (s, 4H, H-4, H-5, H-6, H-7)
7.23 (s, 1H, H-2')
6.90 (m, 2H, H-5', H-6')
5.99 (s, 2H, CH2)

### -exemple 30, 2-[4-(2-Fluorophényl)-semicarbazono]-indane-1,3-dione

12.70 (s, 1H, NH)
10,30 (s, 1H, NH)
8.04 (m, 1H, H-3')
7.97 (s, 4H, H-4, H-5, H-6, H-7)
7.20 (m, 3H, PHe)

### -exemple 31, 2-[4-(3-Fluorophényl)-semicarbazono]-indane-1,3-dione

12.50 (s, 1H, NH)
10,56 (s, 1H, NH)
7.98 (s, 4H, H-4, H-5, H-6, H-7)
7.57 (d, 1H, H-6', J H5'-6' = 10.25 Hz)
7.41 (m, 2H, H-2', H-4')
7.30 (m, 1H, H-5')

### -exemple 32, 2-[4-(4-Fluorophényl)-semicarbazono]-indane-1,3-dione

12.30 (s, 1H, NH)
10,41 (s, 1H, NH)
7.98 (s, 4H, H-4, H-5, H-6, H-7)
7.60 (m, 2H, H-3', H-5')
7.20 (m, 2H, H-2', H-6')

### -exemple 33, 2-[4-(2-Bromophényl)-semicarbazono]-indane-1,3-dione

12.47 (s, 1H, NH)
9.70 (s, 1H, NH)
7.98 (s, 4H, H-4, H-5, H-6, H-7)
7.84 (d, 1H, H-3', J H3'-4' = 8.3 Hz)
7.68 (d, 1H, H-6', J H5'-6' = 8.3 Hz)
7.43 (t, 1H, H-5', J H5'-6' et 4'-6' = 8.3 Hz)
7.16 (t, 1H, H-4', J H3'-4' et H54'-5' = 8.3 Hz)

### -exemple 34, 2-[4-(4-Bromophényl)-semicarbazono]-indane-1,3-dione

12.44 (s, 1H, NH)
10.36 (s, 1H, NH)
7.96 (s, 4H, H-4, H-5, H-6, H-7)
7.53 (s, 4H, H-2',H-3',H-5', H-6')

### -exemple 35, 2-[4-(2-Hydroxyéthyl)-semicarbazono]-indane-1,3-dione

12.11 (s, 1H, NH)
10.20 (s, 1H, NH)
7.96 (s, 5H, H-4, H-5, H-6, H-7, NH)
4.78 (t, 1H, OH)
3.47 (m, 2H, CH2-N)
3.27 (m, 2H, CH2-O)

### -exemple 36, 2-[4-(2-Acétoxyphényl)-semicarbazono]-indane-1,3-dione

12.51 (s, 1H, NH)
9.78 (s, 1H, NH)
7.97 (s, 4H, H-4, H-5, H-6, H-7)
7.80 (m, 1H, H-3')
7.28 (m, 3H, H-4', H-5', H-6')
2.38 (s, 3H, CH3)

### -exemple 37, 5-méthoxy-2-(4-phénylsemicarbazono)-indane-1,3-dione

12.45 (s, 1H, NH)
10.26 (s, 1H, NH)
7.90 (d, 1H, H-7, J H6-7 = 7.8 Hz)
7.58 (d, 1H, H-6, J H6-7 = 7.8 Hz)
7.38 (m,-5H, H-2', H-3', H-5', H-6', H4)
7.07 (m, 1H, H-4')
3.99 (s, 3H, OCH3)

### -exemple 38, 5-Méthoxy-2-(4-phénylthiosemicarbazono)-indane-1,3-dione

12.85 (1s, 1H, NH)
11.17 (s, 1H, NH)
7.96 (d, 1H, H-7, J H6-7 = 7.8 Hz)
7.59(d, 1H, H-6, J H6-7 = 7.8 Hz)
7.45(m, 4H, Ph)
7.27 (m, 1H, Ph)
3.99 (s, 3H, OCH3)

### -exemple 39, 5-méthoxy-2-semicarbazono-indane-1,3-dione

11.94 (s, 1H, NH)
7.90 (d, 1H, H-7, J H6-7 = 8.3 Hz)
7.87 (m, 4H, H-4, H-6, NH2)
3.98 (s, 3H, CH3O)

### -exemple 40, 5-méthoxy-2-[4-(2-Hydroxyphényl)-semicarbazono]-indane-1,3-dione

12.50 (s, 1H, OH)
10.11 (s, 1H, NH)
9.53 (s, 1H, NH)
7.95 (m, 2H, H-7, H-3')
7.45 (m, 2H, H-4, H-6)
6.85 (m, 3H, H-4', H-5', H-6')
3.99 (s, 3H, CH3O)

### -exemple 41, 5-méthoxy-2-thiosemicarbazono-indane-1,3-dione

12.50 (1s, 1H, NH)
12.41 (s, 1H, SH
9.38 (s, 1H, NH)
7.95 (d, 1H, H-7, J H6-7 = 7.8 Hz)
7.47 (m, 2H, H-4, H-6)
3.98 (s, 3H, CH3O)

### -exemple 42, 5-Hydroxy-2-(4-phénylsemicarbazono)-indane-1,3-dione

12.30 (1s, 1H, NH)
11.70 (1s, 1H, NH)
10.29 (1s, 1H, OH)
7.87 (d, 1H, H-6, JH5-6 = 8.3 Hz)
7.60 (d, 1H, H-5, JH5-6 = 8.3 Hz)
7.35 (m, 3H, H-4, H-2', H-6')
7.28 (m, 2H, H-3', H-5')
7.18 (m, 1H, H-4')

### -exemple 43, 5-Hydroxy-2-semicarbazono-indane-1,3-dione

11.90 (1s, 1H, NH)
7.84 (d, 1H, H-7, J H6-7 = 8.3 Hz)
7.30 (m, 4H, H-4, H-6, NH2)

### -exemple 44, 5-Hydroxy-2-(4-phénylthiosemicarbazono)-indane-1,3-dione

11.96 (s, 1H, NH)
11.12 (s, 1H, NH)
7.84 à 7.20 (m, 8H, Ph)

### -exemple 45, 5-Hydroxy-2-thiosemicarbazono-indane-1,3-dione

12.55 (1s, 1H, NH)
12.42 (s,1H, OH)
9.36 (s, 1H, SH)
8.78 (s, 1H, NH)
7.89 (d, 1H, H-7, J H6-7 = 8.3 Hz)
7.27 (m, 2H, H-4, H-6)

### -exemple 46, 5,6-Diméthoxy-2-semicarbazono-indane-1,3-dione

11.78 (s,1H, NH)
7.39 (s, 1H, H-4)
7.35 (s, 1H, H-7)
7.26,(s, 2H, NH2)
3.98 (s, 6H, 2CH3)

### -exemple 47, 5,6-Diméthoxy-2-(4-phénylsemicarbazono)-indane-1,3-dione

12.30 (s,1H, NH)
10.18 (s,1H, NH)
7.59 (m, 7H, H-4, H-7, Ph)
3.99 (s, 6H, 2CH3)

### -exemple 48, 2-[4-(3-Trifluorométhylphényl)-semicarbazono]-indane-1,3-dione

12.50 (1s,1H, NH)
10.68 (s,1H, NH)
8.05 (s, 1H, H2')
7.99 (s, 4H, H-4, H-5, H-6, H-7)
7.83 (d, 1H, H-4', J H4'-5' = 7.8 Hz)
7.60 (t, 1H, H-5', J H4'-5' = et JH5'-6' = 7.8Hz)
7.44 (d, 1H, H-6', J H5'-6' = 7.8Hz)

### -exemple 49, 2-[4-(2-Trifluorométhylphényl)-semicarbazono]-indane-1,3-dione

12.57 (1s,1H, NH)
9.85 (s,1H, NH)
7.99 (s, 4H, H-4, H-5, H-6, H-7)
7.80 (m, 3H, H-3', H-5', H-6')
7.74 (m, 1H, H-3')

### -exemple 50, 2-[4-(3-Bromophényl)-semicarbazono]-indane-1,3-dione

12.44 (s,1H, NH)
10.48 (s,1H, NH)
7.93 (s, 4H, H-4, H-5, H-6, H-7)
7.87 (s, 1H, H-2')
7.45 (d, 1H, H-4', JH4'-5'- = 6.3Hz)
7.25 (m, 2H, H-5', H-6')

### -exemple 51, 2-[4-(4-Bromophényl)-thiosemicarbazono]indane-1,3-dione

12.88 (s,1H, NH)
10.48 (s,1H, NH)
8.02 (s, 4H, H-4, H-5, H-6, H-7)
7.60 (s, 4H, H-2', H-3', H-5', H-6')

### -exemple 63, 1,2-(4-Phénylsemicarbazono)-3-indanone

11.80 (s, 2H, NH)
9.80 (s, 2H, NH)
7.97 (m, 2H, H-4, H-7)
7.65 (m, 6H, H-5, H-6, 2H-2', 2H-6')
7.56 (m, 4H, 2H-3', 2H-5')
7.16 (m, 2H, 2H-4')

### -exemple 64, 1-Oximino-2-(4-phénylsemicarbazono)-3-indanone

13.20 (s, 1H, NH)
11.65 (s, 1H, OH)
9.75 (s, 1H, NH)
8.56 (d, 1H, H-7, J H6-H7 = 7.14 Hz)
7.80 (m, 3H, H-4, H-5, H-6)
7.40 (m, 2H, h-3', H-5')
7.17 (m, 1H, H-4')

### -exemple 65, 1,3-Dioximino-2-(4-phénylthiosemicarbazono)-3-indanone

13.06 (1s, 1H, NH)
12.59 (1s, 1H, OH)
11.63 (1s, 1H, OH)
9.19 (1s, 1H, NH)
8.56 (d, 1H, H-7, H-4, J H6-7 et J H4-5 = 7.3 Hz)
7.64 (m, 4H, H-5, H-6, H-2', H-6')
7.33 (t, 2H, H-3', H-5', J H2'-3' et JH5'-6' = 7.3 Hz)
7.03 (t, 1H, H-4', J H3'-4' et JH4'-5' = 7.3 Hz)

L'étude des composés selon l'invention a montré qu'ils possèdent diverses propriétés pharmacologiques. Ainsi, ils sont veinotoniques et n'affectent pas dans la plupart des cas le système artériel ; par ailleurs, ils augmentent la résistance capillaire, diminuent l'hyperperméabilité vasculaire induite par certains agents inflammatoires et présentent des propriétés antilipoperoxydantes, antiradicalaires et antiinflammatoires, ainsi qu'une activité dans le choc septique.

Ces propriétés sont démontrées chez les mammifères tels que les rats, cobayes et lapins, dans des conditions in vitro (vaisseaux ou réseaux vasculaires isolés) et in vivo.

Pour l'étude in vitro, les composés sont solubilisés en solution aqueuse pure ou contenant du DMSO ou de l'alcool.

Pour l'étude in vivo, ils sont administrés par voie intra-veineuse sous forme de solution aqueuse pure, par voie intrapéritonéale sous forme de solution aqueuse contenant ou non du DMSO ou par voie orale en solution ou en suspension dans la carboxyméthylcellulose ou dans une solution aqueuse composée contenant du Tween^{R} et dans certains cas du DMSO.

### Modèles d'études pharmacologiques

L'effet contractile est mesuré in vitro :
- par la force de contraction développée par des anneaux vasculaires quiescents ou stimulés (soit électriquement soit par des agents physiologiques) et maintenus dans des conditions isométriques,
- par la pression développée par des réseaux vasculaires perfusés à débit constant.

In vivo, les pressions artérielles et veineuses sont mesurées dans des conditions normales et après arrêt cardiaque. Lors de l'arrêt cardiaque, le tonus veineux est calculé à partir des pressions veineuses et artérielles mesurées à l'équilibre et corrigées en fonction des différences relatives de compliance entre ces deux réseaux (Samar et Coleman, Am. J. Physiol., 1978, 234:H94-100 ; Yamamoto et col., Am. J. Physiol., 1980, 238:H823-828).

L'augmentation de la résistance capillaire est appréciée par la modification de l'index pétéchial (pression négative induisant l'extravasation d'érythrocytes), mesuré par une méthode dérivée de l'angiosterromètre de Parrot.

La perméabilité vasculaire est étudiée in vivo et in vitro par la mesure de l'extravasation d'albumine ou de colorant liant l'albumine (Bleu Evans). In vivo, l'hyperperméabilité est induite par l'injection d'une solution d'histamine, de bradykinine ou de zymosan. Les modèles in vitro permettent de réaliser des hyperpressions (sur un territoire vasculaire isolé) et/ou des réactions vasculaires inflammatoires.

L'activité antiinflammatoire est démontrée par la mesure de l'inhibition de l'oedème et de la migration leucocytaire après induction d'une pleurésie chez le rat par injection de carragénine dans la cavité pleurale (Almeida et col., J, Pharmacol. Exp. Therap., 1980, 214:74).

L'effet "piégeur de radicaux libres" global est étudié in vitro par un modèle utilisant le 1,1-diphényl-2-picrylhydrazyl (DPPH) comme radical libre stable, méthode dérivée de celle décrite par Lamaison et col., Plantes Médic. et phytothérapie, XXII, 1988, 231-234.

L'activité antioxydante est étudiée in vitro par un modèle de peroxydation lipidique basée sur la peroxydation d'une émulsion d'acide linoléique par le fer, méthode modifiée par rapport à celle décrite par Sutherland et col., Arch. Biochem. Biophys., 1982, 214,1-11.

L'activité dans le choc septique est étudiée chez le rat après induction par une endotoxine lipopolysaccharidique (15 mg/kg), méthode voisine de celle décrite par Terashita et col., Eur. J. Pharmacol., 109, 257-261, 1985.

### Exemples d'effets pharmacologiques

Les composés de l'invention augmentent la contraction des veines saphènes animales produite par la noradrénaline et la dépolarisation (solution hyperpotassique) sans affecter, dans la majorité des cas, les réponses contractiles artérielles. Ainsi, les composés des exemples 3, 5 et 32 (10 nM à 30 µM) augmentent de plus de 50 % (DE₅₀ ± 0,3 microM) les contractions des veines saphènes de lapins produites par le KCl (40 mM).

Le composé de l' exemple 32 augmente à sa concentration maximale de 30 à 200 % la concentration des veines saphènes de lapin en réponse à la noradrénaline 0,3 micromolaire.

Les composés objet des exemples 3 et 32 augmentent de plus de 20 % le tonus veineux de base du rat sans affecter la pression artérielle à des doses de 1 à 3 picomoles en administration i.v.

A titre illustratif, le composé de l'exemple 4 augmente la résistance capillaire de base de 10 à 100 %, lorsque celle-ci est mesurée une heure à deux heures après administration de 5-20 mg/kg/i.p. et jusqu'à quatre à six heures après administration orale de 5-20 mg/kg chez le rat.

Le composé de l' exemple 8 diminue la perméabilité vasculaire de 10 à 50 %, lorsque celle-ci est mesurée une à deux heures après administration de 5-20 mg/kg/i.p. et deux à quatre heures après administration orale de 5-20 mg/kg chez le rat.

Le composé de l'exemple 3 administré deux fois en i.p. à la dose de 20 mg/kg inhibe l'oedème et la migration leucocytaire dans la cavité pleurale, 6 heures après injection de carragénine dans le modèle de la pleurésie chez le rat.

Le composé de l'exemple 1 présente un effet antiradicalaire maximal de plus de 95 % dans le modèle utilisant le DPPH.

Le composé de l'exemple 3, à des doses i.v. aussi faibles que 1 microgramme/kg, réduit de plus de 30 % la chute de pression artérielle initiale produite par l'endotoxine et restaure cette pression artérielle après 30 minutes, alors qu'elle reste diminuée de 30 % chez les rats témoins.

### Toxicité

Par ailleurs, les composés de l'invention sont très peu toxiques. Par exemple, après administration unique per os chez la souris, aucune mortalité n'est observée à la dose de 1 g/kg avec les composés des exemples 3 et 32. Les seuls effets observés sont dans certains cas des diarrhées colorèes et des urines colorées, ces dernières étant le témoin d'une résorption du produit.

Ce qui précède montre que les composés selon l'invention peuvent être utilisés en thérapeutique humaine et animale. Ils sont en particulier indiqués dans l'insuffisance veineuse fonctionnelle, organique et les pathologies hémorroïdaires par leurs composantes vasculaires et antiinflammatoires, ainsi que dans les affections typiquement inflammatoires (ostéoarticulaires, dermatologiques ou cardiovasculaires) et dans les états de chocs constitués par une chute importante de la pression artérielle, en particulier dans les états de chocs septiques (endotoxiques).

L'insuffisance veineuse fonctionnelle se caractérise par une dilatation et une hyperdistensibilité des veines superficielles des membres inférieurs accompagnées par des symptômes fonctionnels : douleurs des membres inférieurs, oedèmes, paresthèsies à type d'impatiences, de jambes sans repos. Ce type de pathologie peut évoluer vers l' insuffisance veineuse organique (varices, incontinence valvulaire profonde ...) voire vers la phlébothrombose et les lésions ulcéreuses.

Dans cette pathologie veineuse, une composante inflammatoire s installe dans les premiers stades et se manifeste plus clairement dans les stades avancés.

La présente invention comprend donc l'utilisation des composés ci-dessus décrits, comme substance active pour la préparation de médicaments et compositions pharmaceutiques, à usage humain ou vétérinaire, comprenant au moins un desdits composés en association avec un support ou diluant physiologiquement acceptable.

La forme de ces médicaments et compositions pharmaceutiques dépendra bien évidemment de la voie d'administration souhaitée notamment orale, parentérale et rectale et ils peuvent être formulés selon les techniques classiques avec mise en oeuvre des supports et véhicules usuels.

Ainsi, dans le cas d'une administration par voie orale, ils peuvent se présenter sous la forme de comprimés, tablettes, gélules, solutions, sirops et suspensions.

Les comprimés, tablettes et gélules contiennent la substance active conjointement avec un diluant (par exemple lactose, dextrose, sucrose, mannitol, sorbitol ou cellulose), un lubrifiant (par exemple silice, talc ou stéarate comme le stéarate de magnésium), un liant (par exemple amidon, méthylcellulose ou gomme arabique), un agent de désintégration (alginate par exemple) et ils sont fabriqués par des techniques connues par exemple de mélange, de granulation de pastillage, d'enrobage, etc...

Les sirops peuvent contenir, à titre de support, glycérol, mannitol et/ou sorbitol. Les solutions et suspensions peuvent comprendre de l'eau et un support tel qu'une gomme naturelle, de la gélose, de l'alginate de sodium ou de l'alcool polyvinylique.

Pour l'administration par voie parentérale, les médicaments et compositions peuvent prendre la forme de solutions, d'émulsions ou de suspensions comprenant la substance active et un support approprié tel que l'eau stérile ou des solutions salines isotoniques aqueuses stériles.

Pour l'administration par voie rectale, ils peuvent prendre la forme de suppositoires comprenant la substance active et un support approprié tel que le beurre de cacao ou du polyéthylène glycol.

La dose thérapeutique des substances actives pourra atteindre 1000 mg/jour suivant la voie d'administration, l'âge, le poids et l'état du sujet à traiter et la puissance thérapeutique de la substance active mise en oeuvre.

## Revendications

1. Composés répondant à la formule : 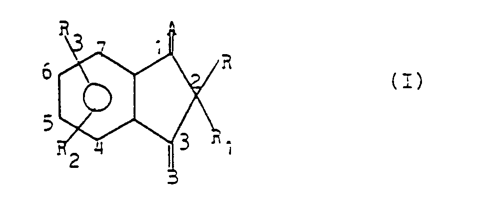 dans laquelle R₂ et R₃ représentent indépendamment l'un de l'autre H, alkoxy en C₁-C₄ ou OH ; et le couple (A, B) prend la valeur :
- (oxygène, oxygène), auquel cas l'un parmi R et R₁ représente OH, halogène, (alkyl en C₁-C₄)NH, N-morpholino(alkyl en C₁-C₄)NH, 1-(pyridyl)-4-pipérazino ou 1-(phényl)-4-pipérazino dont le noyau phényle est éventuellement substitué par un halogène et l'autre représente un groupe NHNHCONHR₄ où R₄ = phényle ou phényle substitué par OH ou alkyle en C₁-C₄.
R et R₁ pouvant par ailleurs former ensemble un groupe :
. =N-NH-CX-NHR₅ où X représente oxygène ou soufre et R₅ = H, pyridyle, phényle, ou phényle substitué par un, deux ou trois groupes choisis parmi OH, CF₃, alkyle en C₁-C₄, alkoxy en C₁-C₄, halogène, méthylènedioxy, acétoxy et hydroxyéthyle,
. =NNHCXN(phényle)₂, où X représente oxygène ou soufre,
. =N-NH-CX-NH-NH-R₅ où X et R₅ ont les mêmes significations que ci-dessus, ou
. =N-NH-C(SCH₃)=NR₆ ou =N-N=C(SCH₃)-NHR₆ où R₆ représente phényle ou phényle substitué par un, deux ou trois groupes choisis parmi OH, CF₃, alkyle en C₁-C₄, alkoxy en C₁-C₄, halogène, méthylènedioxy, acétoxy et hydroxyéthyle ;
- (N-OH, oxygène), auquel cas R et R₁ forment ensemble un groupe =NNHCXNHR₅ ou =NNHCXN(phényle)₂ où X et R₅ ont les mêmes significations que ci-dessus :
- (NNHCXNHR₅, oxygène) où X et R₅ ont les mêmes significations que ci-dessus, auquel cas R et R₁ forment ensemble un groupe =NNHCXNHR₅ ; ou
- (N-OH, N-OH), auquel cas R et R₁ forment ensemble un groupe =NNHCXNHR₅ où X et R₅ ont les mêmes significations que ci-dessus ou
un groupe =N-NH-CX-N(phényle)₂.
ainsi que les sels d'addition d'acide des composés salifiables parmi ceux de formule (I) ci-dessus .

2. Composés selon la revendication 1, caractérisés en ce qu'ils répondent à la formule : 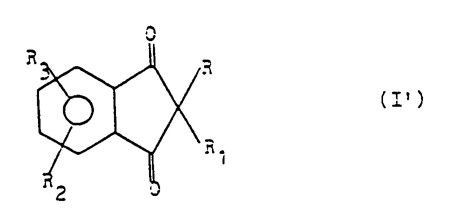 où R, R₁, R₂ et R₃ ont les mêmes significations que dans la formule (I) quand le couple (A, B) y prend la valeur (oxygène, oxygène),

3. Composés selon la revendication 1, caractérisés en ce qu'ils répondent à la formule : 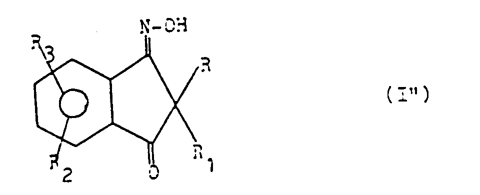 où R, R₁, R₂ et R₃ ont les mêmes significations que dans la formule (I) quand le couple (A, B) y prend la valeur ( N-OH, oxygène).

4. Composés selon la revendication 1, caractérisés en ce qu'ils répondent à la formule : 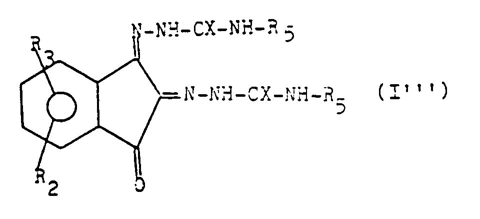 où R₂ et R₃, X et R₅ ont les mêmes significations que dans la formule (I).

5. Composés selon la revendication 1, caractérisés en ce qu'ils répondent à la formule : 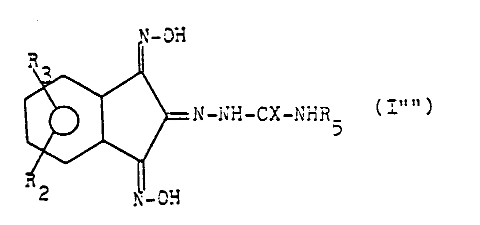 où R₂ et R₃, X et R₅ ont les mêmes sigifications que dans la formule (I).

6. Composés selon la revendication 1, caractérisés en ce qu'ils répondent à l'une des formules (Ia)-(Im) suivantes :
(a) 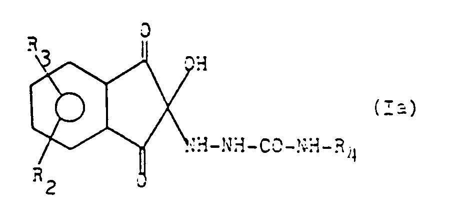
(b) 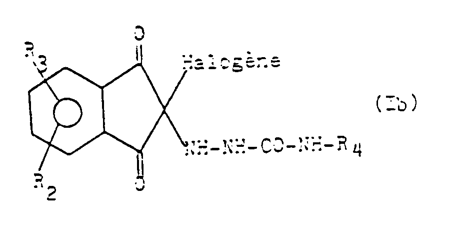
(c) 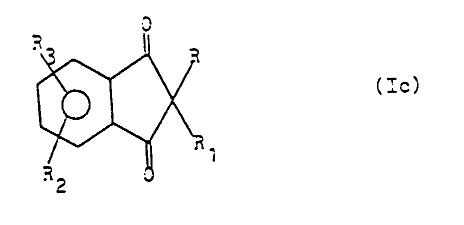 où l'un parmi R et R₁ représente NHNHCONHR₄ et l'autre représente (alkyl en C₁-C₄)NH, N-morpholino (alkyl en C₁-C₄)NH, 1-(pyridyl)-4-pipérazino ou 1-(phényl)-4-pipérazino dont le noyau phényle est éventuellement substitué par un halogène.
(d) 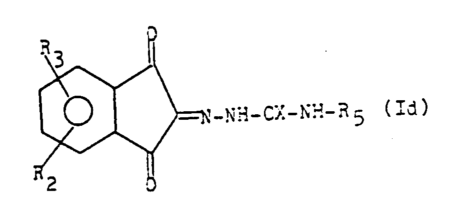
(e) 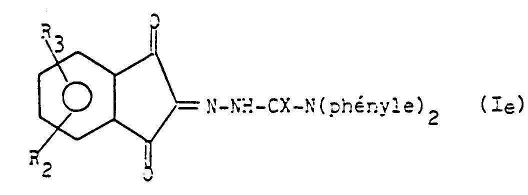
(f) 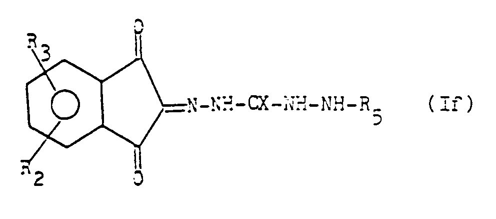
(g) 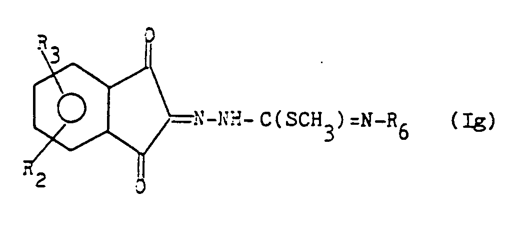
(h) 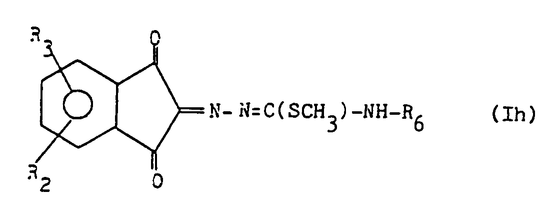
(i) 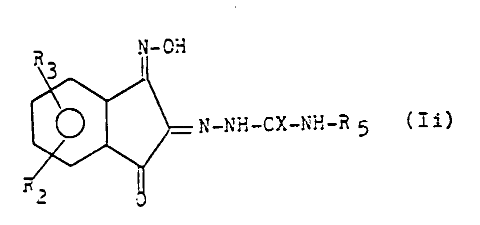
(j) 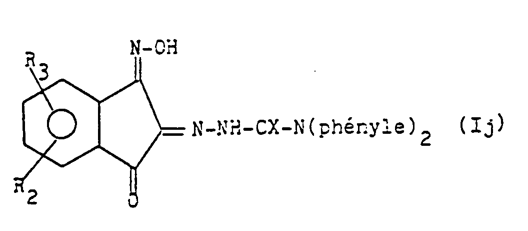
(k) 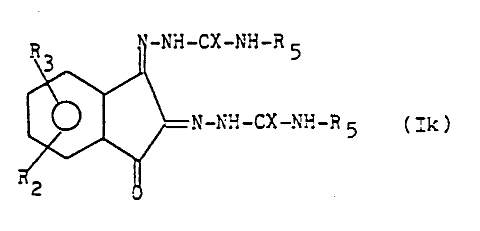 (l) 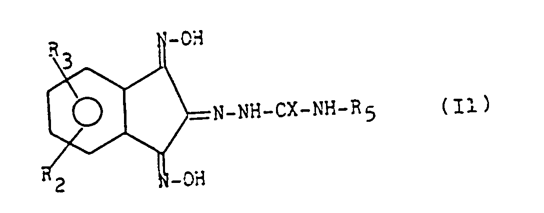
(m) 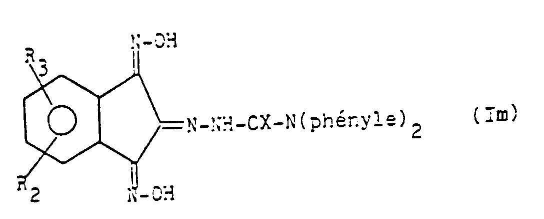 les symboles R₂, R₃, R₄, R₅ et X ayant les mêmes significations que dans la formule (I).

7. Utilisation des composés selon l'une des revendications 1 à 4, pour la fabrication d'un médicament.

8. Composition pharmaceutique, caractérisée en ce qu'elle comprend au moins un composé ou sel pharmaceutiquement acceptable de ce composé, selon l'une des revendications 1 à 6 et un support ou diluant physiologiquement acceptable.

9. Procédé de préparation des composés de formule (I) selon la revendication 1, pour lesquels le couple (A, B) = (oxygène, oxygène), l'un parmi R et R₁ représente OH ou halogène et l'autre représente NHNHCONHR₄, caractérisé en ce qu'il comprend la condensation des composés de formule :
H₂N-NH-CO-NH-R₄
sur la ninhydrine de formule : 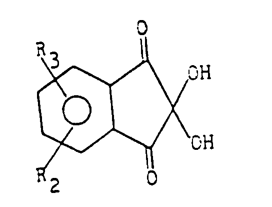 R₂ ,R₃ et R₄ ayant la même signification que dans la revendication 1, éventuellement suivie de la réaction des composés ainsi obtenus, avec un agent halogénant.

10. Procédé de préparation des composés de formule (I) selon la revendication 1, pour lesquels le couple (A, B) = (oxygène, oxygène), l'un parmi R et R₁ représente (alkyle en C₁-C₄), N-morpholino (alkyl en C₁-C₄)NH, 1-(pyridyl)-4-pipérazino ou 1-(phényl)-4-pipérazino dont le noyau phényle est éventuellement substitué par un halogène et l'autre représente NHNHCONHR₄, caractérisé en ce qu'il comprend la condensation de (alkyl en C₁-C₄)NH₂, N-morpholino (alkyl en C₁-C₄)NH₂, 1-(pyridyl)-4-pipérazine ou 1-(phényl)-4-pipérazine dont le noyau phényle est éventuellement substitué par un halogène, respectivement sur les composés de formule : 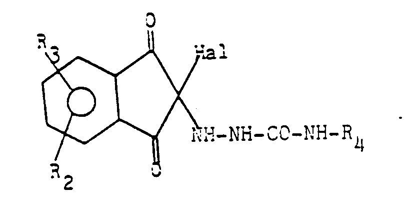 où R₂, R₃ et R₄ ont la même signification que dans la revendication 1 et Hal = halogène.

11. Procédé de préparation des composés de formule (I) selon la revendication 1, pour lesquels le couple (A, B) = (oxygène, oxygène) et R et R₁ forment ensemble un groupe =N-NH-CX-NH-R₅ ou =N-NH-CX-N(phényle)₂, caractérisé en ce qu'il comprend la condensation du chlorhydrate de H₂N-NH-CX-NHR₅ ou de H₂N-NH-CX-N(phényle)₂ sur la ninhydrine de formule : 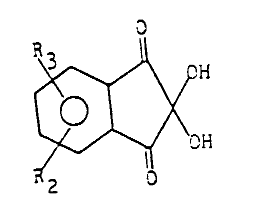 R₂, R₃, X et R₅ ayant la même signification que dans la revendication 1.

12. Procédé de préparation des composés de formule (I), selon la revendication 1, pour lesquels le couple (A, B) = (oxygène, oxygène) et R et R₁ forment ensemble un groupe =N-NH-CX-NH-NH-R₅, caractérisé en ce qu'il comprend la condensation de H₂N-NH-CX-NH-NH-R₅ sur la ninhydride de formule : 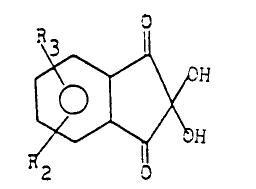 où R₂, R₃, X et R₅ ont la même signification que dans la revendicatin 1, dans l'éthanol en faisant suivre par un traitement par HCl dans l'éthanol.

13. Procédé de préparation des composés de formule (I), selon la revendication 1, pour lesquels le couple (A, B) = (oxygène, oxygène) et R et R₁ forment ensemble un groupe =N-NH-C(SCH₃)=N-R₆ ou =N-N=C(SCH₃)-NH-R₆, caractérisé en ce qu'il comprend la condensation à chaud de H₂N-NH-C(SCH₃)=N-R₆ sur la ninhydrine de formule : 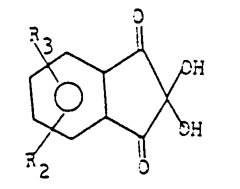 R₂, R₃ et R₆ ayant la même signification que dans la revendication 1.

14. Procédé de préparation des composés de formule (I), pour lesquels le couple (A, B) = (N-OH, oxygène) ou (N-OH, N-OH) et R et R₁ forment ensemble un groupe =N-NH-CX-NH-R₅ ou =N-NH-CX-N(phényle)₂, caractérisé en ce qu'il comprend la condensation à chaud d'un ou deux équivalents de chlorhydrate d'hydroxylamine sur les composés de formule (I), selon la revendication 1, pour lesquels le couple (A, B) = (oxygène, oxygène) et R et R₁ ont la même signification que ci-dessus.

15. Procédé de préparation des composés de formule (I), selon la revendication 1, pour lesquels le couple (A, B) = (N-NH-CX-NH-R₅, oxygène) et R et R₁ forment ensemble un groupe =N-NH-CX-NH-R₅, caractérisé en ce qu'il comprend la condensation de deux équivalents de H₂N-NH-CX-NH-R₅ sur la ninhydride de formule : 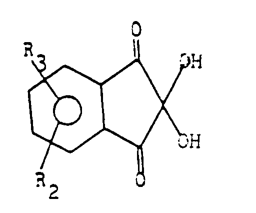 R₂, R₃ et R₅ ayant la même signification que dans la revendicatin 1.

## Claims

1. Compounds having the formula: 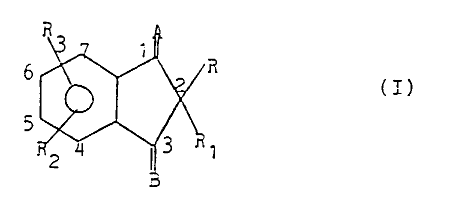 in which R₂ and R₃ independently denotes H, C₁-C₄ alkoxy or OH; and the pair (A, B) denotes :
- (oxygen, oxygen), in which case one out of R and R₁ denotes OH, halogen, (C₁-C₄ alkyl) NH, N-morpholino(C₁-C₄ alkyl) NH, 1-(pyridyl)-4-piperazino or 1-(phenyl)-4-piperazino in which the phenyl ring is optionally substituted by a halogen, and the other denotes an NHNHCONHR₄ group where R₄ = phenyl or phenyl substituted by OH or C₁-C₄ alkyl and
R and R₁ may also together form a group:
. =N-NH-CX-NHR₅ where X represents oxygen or sulphur and R₅ = H, pyridyl, phenyl or phenyl substituted by one, two or three groups chosen from among OH, CF₃, C₁-C₄ alkyl, C₁-C₄ alkoxy, halogen, methylene dioxy, acetoxy or hydroxyethyl,
. =NNHCXN (phenyl)₂, where X denotes oxygen or sulphur,
. =N-NH-CX-NH-NH-R₅ where X and R₅ have the same meanings as hereinbefore, or
. =N-NH-C(SCH₃)=NR₆ or =N-N=C(SCH₃)-NHR₆ where R₆ denotes phenyl or phenyl substituted by one, two or three groups chosen from among OH, CF₃, C₁-C₄ alkyl, C₁-C₄ alkoxy, halogen, methylene dioxy, acetoxy or hydroxyethyl;
- (N-OH, oxygen) in which case R and R₁ together form an =NNHCXNHR₅ or =NNHCXN (phenyl)₂ group, where X and R₅ have the same meanings as hereinbefore ;
- (NNHCXNHR₅, oxygen) where X and R₅ have the same meanings as hereinbefore, in which case R and R₁ together form an =NNHCXNHR₅ group ; or
- (N-OH, N-OH), in which case R and R₁ together form an =NNHCXNHR₅ group where X and R₅ have the same meanings as hereinbefore, or an =N-NH-CX-N (phenyl)₂ group,
as well as the acid addition salts of the salt-forming compounds among the formula (I) compounds hereinbefore.

2. Compounds according to claim 1, characterised in that they have the formula: 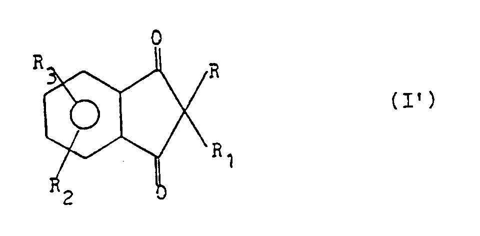 where R, R₁, R₂ and R₃ have the same meanings as in formula (I) when the pair (A, B) therein denotes (oxygen, oxygen).

3. Compounds according to claim 1, characterised in that they have the formula: 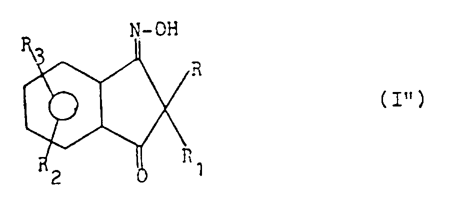 where R, R₁, R₂ and R₃ have the same meanings as in formula (I), when the pair (A, B) therein denotes (N-OH, oxygen).

4. Compounds according to claim 1, characterised in that they have the formula: 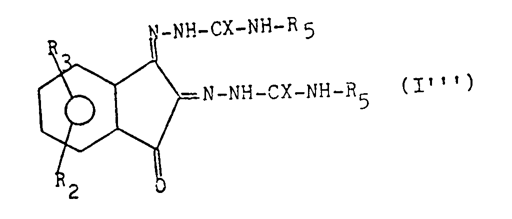 where R₂ and R₃, X and R₅ have the same meanings as in formula (I).

5. Compounds according to claim 1, characterised in that they have the formula: 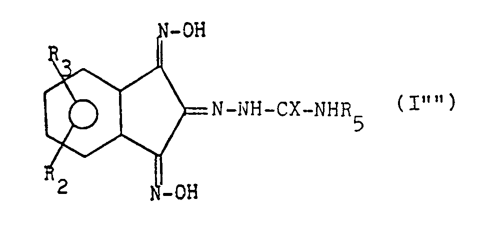 where R₂ and R₃, X and R₅ have the same meanings as in formula (I).

6. Compounds according to claim 1, characterised in that they have one of the following formulae (Ia)-(Im):
(a) 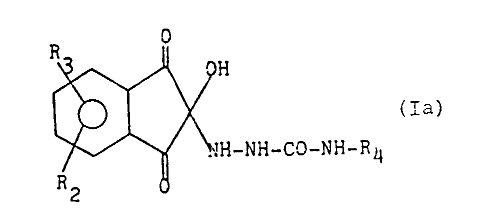
(b) 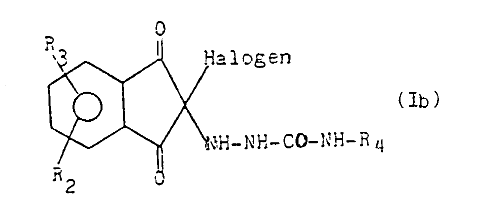
(c) 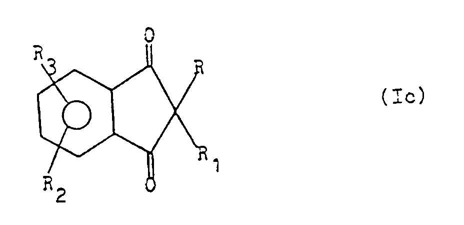 where one out of R and R₁ denotes NHNHCONHR₄ and the other denotes (C₁-C₄ alkyl) NH, N-morpholino (C₁-C₄ alkyl) NH, 1-(pyridyl)-4-piperazino or 1-(phenyl)-4-piperazino in which the phenyl ring is optionally substituted by a halogen,
(d) 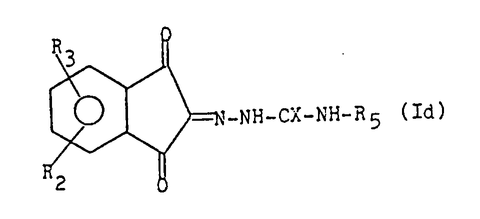
(e) 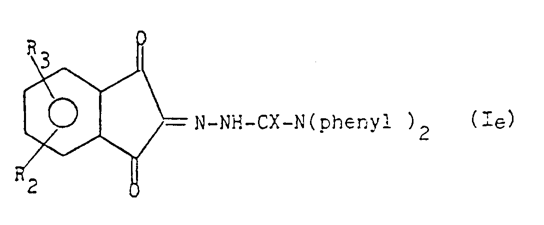
(f) 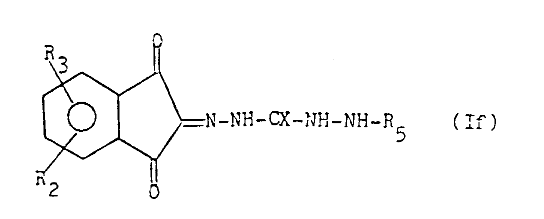
(g) 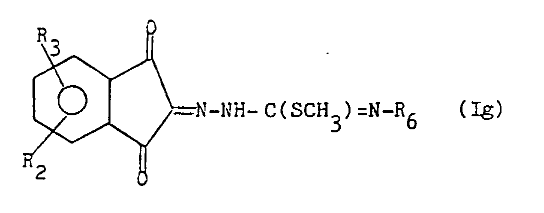
(h) 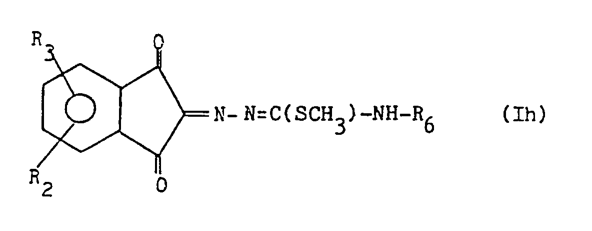
(i) 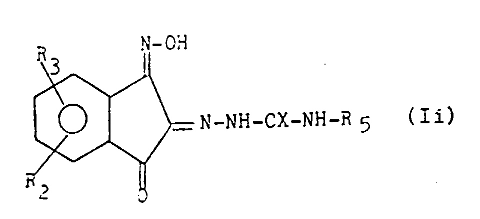
(j) 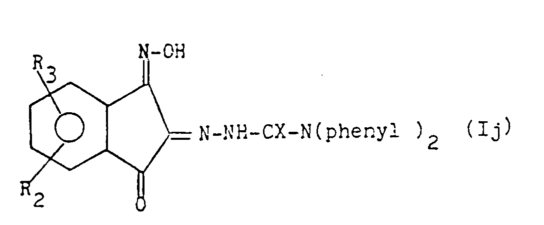
(k) 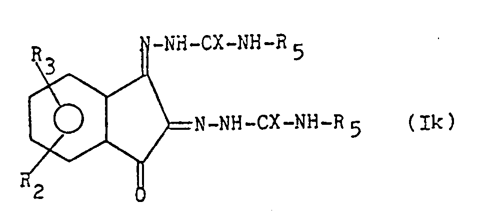
(l) 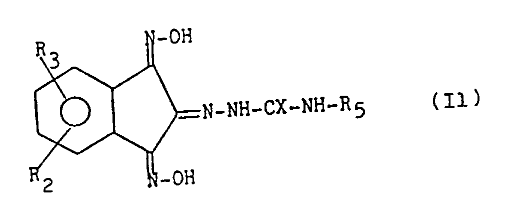
(m) 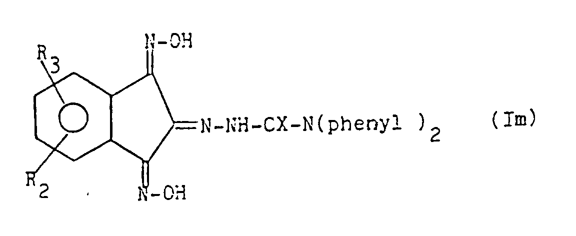 in which the symbols R₂, R₃, R₄, R₅ and X have the same meanings as in formula (I).

7. Use of the compounds according to any of claims 1 to 4 for manufacturing a drug.

8. A pharmaceutical composition, characterised in that it comprises at least one compound or pharmaceutically acceptable salt thereof,according to any of claims 1 to 6 and a physiologically acceptable excipient or diluent.

9. A method of preparing formula (I) compounds according to claim 1, in which the pair (A, B) = (oxygen, oxygen), one out of R and R₁ denotes OH or halogen and the other denotes NHNHCONHR₄, characterised in that it comprises condensation of compounds having the formula:
H₂N-NH-CO-NH-R₄
on ninhydrin having the formula: 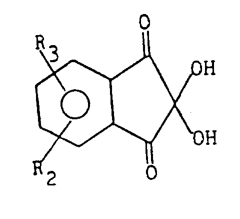 where R₂, R₃ and R₄ have the same meanings as in claim 1, optionally followed by reaction of the resulting compounds with a halogenating agent.

10. A method of preparing formula (I) compounds according to claim 1, in which the pair (A, B) = (oxygen, oxygen), one out of R and R₁ denotes (C₁-C₄ alkyl) NH, N-morpholino (C₁-C₄ alkyl) NH, 1-(pyridyl)-4-piperazino or 1-(phenyl)-4-piperazino in which the phenyl ring is optionally substituted by a halogen and the other denotes NHNHCONHR₄, the method being characterised in that it comprises condensation of (C₁-C₄ alkyl) NH₂, N-morpholino (C₁-C₄ alkyl) NH₂, 1-(pyridyl)-4-piperazine or 1-(phenyl)-4-piperazine in which the phenyl ring is optionally substituted by a halogen, respectively with compounds having the formula: 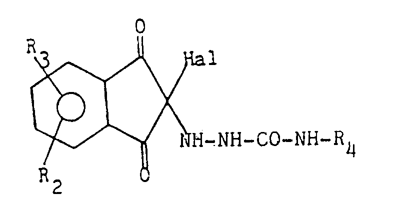 where R₂, R₃ and R₄ have the same meanings as in claim 1 and Hal = halogen.

11. A method of preparing formula (I) compounds according to claim 1, in which the pair (A, B) = (oxygen, oxygen) and R and R₁ together form an =N-NH-CX-NH-R₅ or =N-NH-CX-N (phenyl)₂ group, characterised in that it comprises condensation of H₂N-NH-CX-NHR₅ hydrochloride or H₂N-NH-CX-N(phenyl)₂ hydrochloride with ninhydrin having the formula: 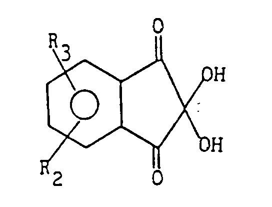 where R₂, R₃, X and R₅ have the same meanings as in claim 1.

12. A method of preparing formula (I) compounds according to claim 1, in which the pair (A, B) = (oxygen, oxygen) and R and R₁ together form an =N-NH-CX-NH-NH-R₅ group, characterised in that it comprises condensation of H₂N-NH-CX-NH-NH-R₅ with the ninhydrin having the formula: 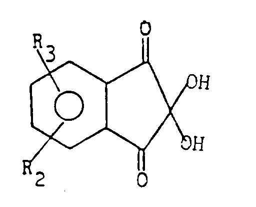 where R₂, R₃, X and R₅ have the same meanings as in claim 1, in ethanol, followed by treatment with HCl in ethanol.

13. A method of preparing formula (I) compounds according to claim 1, in which the pair (A, B) = (oxygen, oxygen) and R and R₁ together form an =N-NH-C(SCH₃)=N-R₆ or =N-N=C(SCH₃)-NH-R₆ group, characterised in that it comprises hot condensation of H₂N-NH-C(SCH₃)=N-R₆ with ninhydrin having the formula: 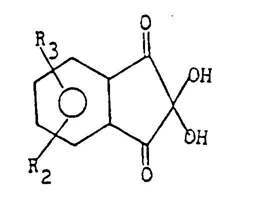 where R₂, R₃ and R₆ have the same meanings as in claim 1.

14. A method of preparing formula (I) compounds in which the pair (A, B) = (N-OH, oxygen) or (N-OH, N-OH) and R and R₁ together form an =N-NH-CX-NH-R₅ or an =N-NH-CX-N(phenyl)₂ group, characterised in that it comprises hot condensation of one or two equivalents of hydroxylamine hydrochloride with formula (I) compounds according to claim 1, in which the pair (A, B) =(oxygen, oxygen) and R and R₁ has the same meaning as hereinbefore.

15. A method of preparing formula (I) compounds according to claim 1, in which the pair (A, B) =(N-NH-CX-NH-R₅, oxygen) and R and R₁ together form an =N-NH-CX-NH-R₅ group, characterised in that it comprises condensation of two equivalents of H₂N-NH-CX-NH-R₅ with the ninhydrin having the formula: 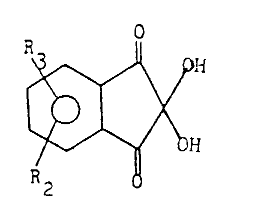 in which R₂, R₃ and R₅ have the same meaning as in claim 1.

## Patentansprüche

1. Verbindungen, entsprechend der Formel: 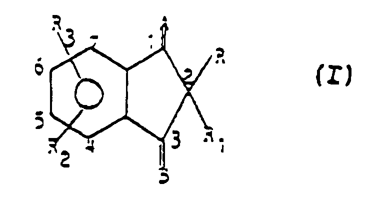 in welcher R₂ und R₃ unabhängig voneinander H, C₁-C₄-Alkoxy oder OH darstellen; und das Paar (A, B) den Wert annimmt:
- (Sauerstoff, Sauerstoff), wobei in diesem Fall eines von R und R₁ OH, Halogen, (C₁-C₄-Alkyl)NH, N-Morpholino(C₁-C₄-alkyl)NH, 1-(Pyridyl)-4-piperazino oder 1-(Phenyl)-4-piperazino, dessen Phenylkern gegebenenfalls mit einem Halogen substituiert ist, darstellt und das andere eine Gruppe NHNHCONHR₄ darstellt, wobei R₄ = Phenyl oder Phenyl, das mit OH oder C₁-C₄-Alkyl substituiert ist.
R und R₁ können außerdem zusammen eine Gruppe bilden:
. =N-NH-CX-NHR₅, wobei X Sauerstoff oder Schwefel darstellt und R₅ = H, Pyridyl, Phenyl oder Phenyl, das mit ein, zwei oder drei Gruppen substituiert ist, die unter OH, CF₃, C₁-C₄-Alkyl, C₁-C₄-Alkoxy, Halogen, Methylendioxy, Acetoxy und Hydroxyethyl ausgewählt sind.
. =NNHCXN(Phenyl)₂, wobei X Sauerstoff oder Schwefel darstellt,
. =N-NH-CX-NH-NH-R₅, wobei X und R₅ dieselben Bedeutungen wie oben haben, oder
. =N-NH-C(SCH₃)=NR₆ oder =N-N=C(SCH₃)-NHR₆, wobei R₆ Phenyl oder Phenyl, das mit einer, zwei oder drei Gruppen, ausgewählt unter OH, CF₃, C₁-C₄-Alkyl, C₁-C₄-Alkoxy, Halogen, Methylendioxy, Acetoxy und Hydroxyethyl, substituiert ist, darstellt;
- (N-OH, Sauerstoff), wobei in diesem Fall R und R₁ zusammen eine Gruppe =NNHCXNHR₅ oder =NNHCXN(Phenyl)₂ bilden, wobei X und R₅ dieselben Bedeutungen haben wie oben;
- (NNHCXNHR₅, Sauerstoff), wobei X und R₅ dieselben Bedeutungen haben wie oben, wobei in diesem Fall R und R₁ zusammen eine Gruppe =NNHCXNHR₅ bilden, oder
- (N-OH, N-OH), wobei in diesem Fall R und R₁ zusammen eine Gruppe =NNHCXNHR₅, wobei X und R₅ dieselben Bedeutungen haben wie oben, oder eine Gruppe =N-NH-CX-N(Phenyl)₂ bilden,
sowie die Säuresalze der salzbildenden Verbindungen unter denen der obigen Formel (I).

2. Verbindungen gemäß Anspruch 1, **dadurch gekennzeichnet**, daß sie der Formel: 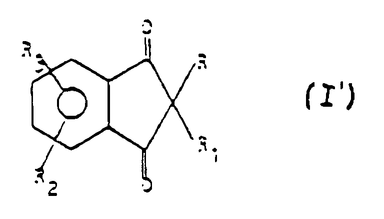 entsprechen, wobei R, R₁ und R₃ dieselben Bedeutungen haben wie in Formel (I), wenn das Paar (A, B) dort den Wert (Sauerstoff, Sauerstoff) annimmt.

3. Verbindungen gemäß Anspruch 1, **dadurch gekennzeichnet**, daß sie der Formel: 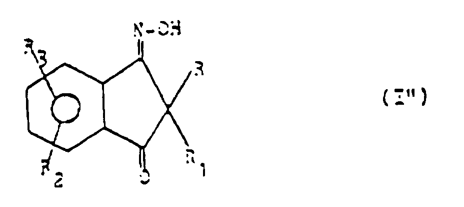 entsprechen, wobei R, R₁, R₂ und R₃ dieselben Bedeutungen haben wie in Formel (I), wenn das Paar (A, B) dort den Wert (N-OH, Sauerstoff) annimmt.

4. Verbindungen gemäß Anspruch 1, **dadurch gekennzeichnet,** daß sie der Formel: 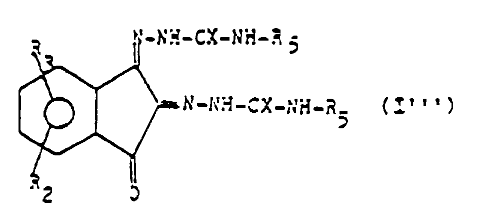 entsprechen, wobei R₂ und R₃, X und R₅ dieselben Bedeutungen haben wie in Formel (I).

5. Verbindungen gemäß Anspruch 1, **dadurch gekennzeichnet**, daß sie der Formel: 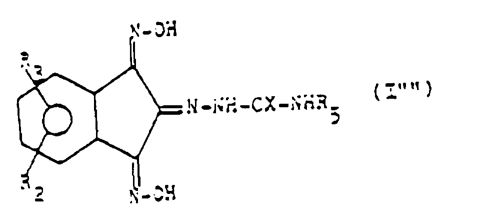 entsprechen, wobei R₂ und R₃, X und R₅ dieselben Bedeutungen haben wie in Formel (I).

6. Verbindungen gemäß Anspruch 1, **dadurch gekennzeichnet**, daß sie einer der folgenden Formeln (Ia)-(Im) entsprechen:
(a) 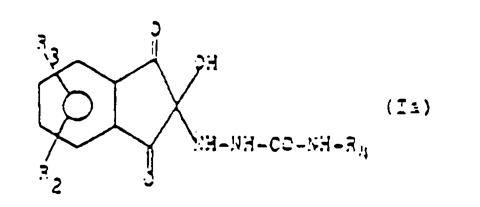
(b) 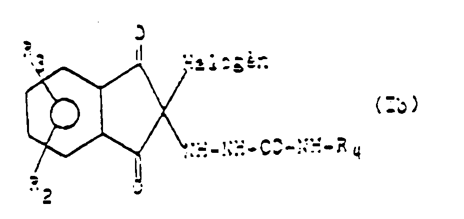
(c) 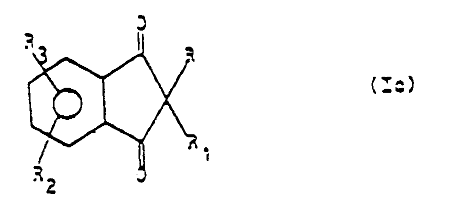 wobei eines von R und R₁ NHNHCONHR₄ darstellt und das andere (C₁-C₄-Alkyl)NH, N-Morpholino(C₁-C₄-alkyl)NH, 1-(Pyridyl)-4-piperazino oder 1-(Phenyl)-4-piperazino, dessen Phenylkern gegebenenfalls mit einem Halogen substituiert ist, darstellt.
(d) 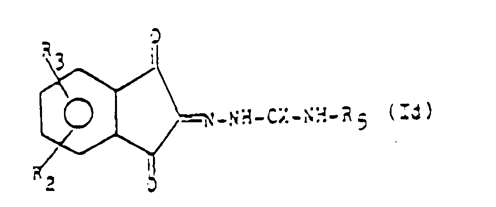
(e) 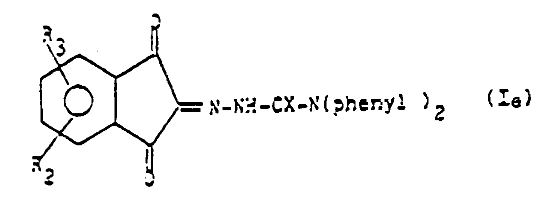
(f) 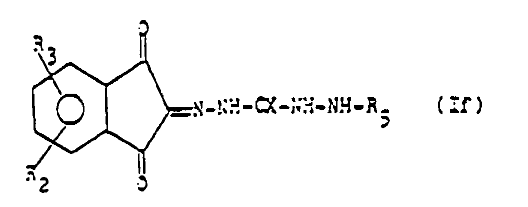
(g) 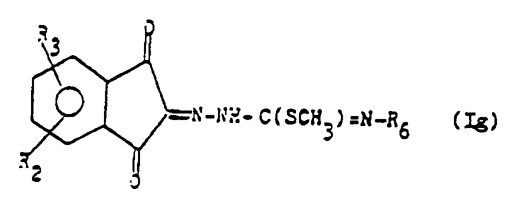
(h) 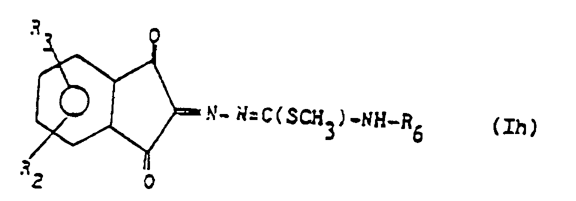
(i) 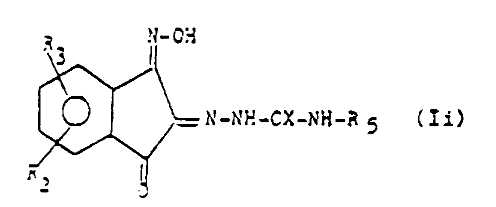
(j) 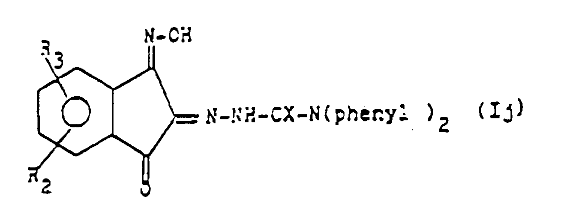
(k) 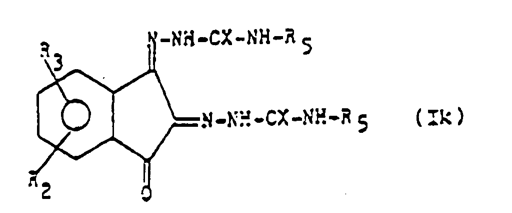
(l) 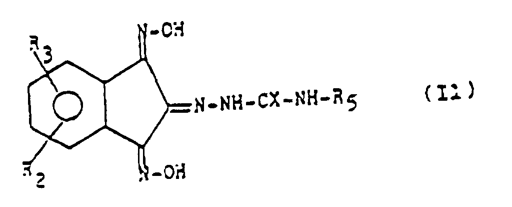
(m) 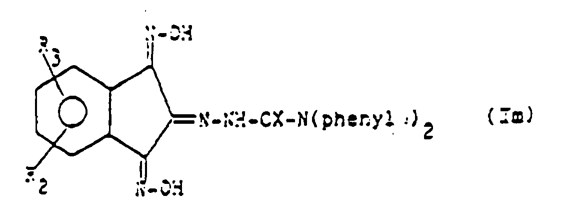 wobei die Symbole R₂, R₃, R₄, R₅ und X dieselben Bedeutungen haben wie in Formel (I).

7. Verwendung der Verbindungen gemäß einem der Ansprüche 1 bis 4, für die Herstellung eines Medikamentes.

8. Pharmazeutische Zusammensetzung, **dadurch gekennzeichne**t, daß sie mindestens eine Verbindung oder ein pharmazeutisch annehmbares Salz dieser Verbindung gemäß einem der Ansprüche 1 bis 6 und einen Trägerstoff oder physiologisch annehmbares Verdünnungsmittel umfaßt.

9. Verfahren zur Herstellung von Verbindungen der Formel (I) gemäß Anspruch 1, für welche das Paar (A, B) = (Sauerstoff, Sauerstoff), wobei eines von R und R₁ OH oder Halogen darstellt und das andere NHNHCONHR₄ darstellt, **dadurch gekennzeichnet**, daß es die Kondensation von Verbindungen der Formel:
H₂N-NH-CO-NH-R₄
mit Ninhydrin der Formel: 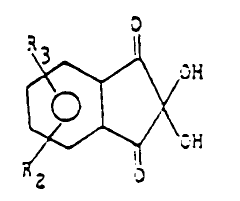 umfaßt, wobei R₂, R₃ und R₄ dieselbe Bedeutung haben wie in Anspruch 1, gegebenenfalls gefolgt von der Reaktion der somit erhaltenen Verbindungen mit einem Halogenierungsmittel.

10. Verfahren zur Herstellung von Verbindungen der Formel (I) gemäß Anspruch 1, für welche das Paar (A, B) = (Sauerstoff, Sauerstoff), wobei eines von R und R₁ (C₁-C₄-Alkyl), N-Morpholino(C₁-C₄-alkyl)NH, 1-(Pyridyl)-4-piperazino oder 1-(Phenyl)-4-piperazino, dessen Phenylkern gegebenenfalls mit einem Halogen substituiert ist, darstellt und das andere NHNHCONHR₄ darstellt**, dadurch gekennzeichnet**, daß es die Kondensation von (C₁-C₄-Alkyl)NH₂, N-Morpholino(C₁-C₄-alkyl)NH₂, 1-(Pyridyl)-4-piperazin oder 1-(Phenyl)-4-piperazin, dessen Phenylkern gegebenenfalls mit einem Halogen substituiert ist, jeweils mit den Verbindungen der Formel: 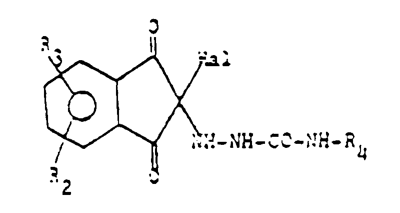 umfaßt, wobei R₂, R₃ und R₄ dieselbe Bedeutung haben wie in Anspruch 1 und Hal = Halogen.

11. Verfahren zur Herstellung von Verbindungen dar Formel (I) gemäß Anspruch 1, für welche das Paar (A, B) = (Sauerstoff, Sauerstoff), und R und R₁ zusammen eine Gruppe =N-NH-CX-NH-R₅ oder =N-NH-CX-N(Phenyl)₂ bilden, **dadurch gekennzeichnet**, daß es die Kondensation von H₂N-NH-CX-NHR₅-Hydrochlorid oder H₂N-NH-CX-N(Phenyl)₂ mit Ninhydrin der Formel: 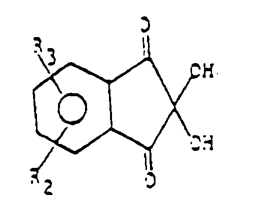 umfaßt, wobei R₂, R₃, X und R₅ dieselbe Bedeutung haben wie in Anspruch 1.

12. Verfahren zur Herstellung von Verbindungen der Formel (I) gemäß Anspruch 1, für welche das Paar (A, B) = (Sauerstoff, Sauerstoff), und R und R₁ zusammen eine Gruppe =N-NH-CX-NH-NH-R₅ bilden, **dadurch gekennzeichnet**, daß es die Kondensation von H₂N-NH-CX-NH-NH-R₅ mit Ninhydrin der Formel: 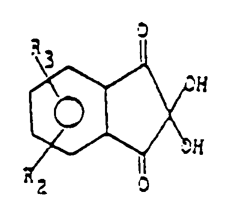 wobei R₂, R₃, X und R₅ dieselbe Bedeutung haben wir in Anspruch 1, in Ethanol umfaßt, worauf eine Behandlung mit HCl in Ethanol folgt.

13. Verfahren zur Herstellung von Verbindungen der Formel (I) gemäß Anspruch 1, für welche das Paar (A, B) = (Sauerstoff, Sauerstoff), und R und R₁ zusammen eine Gruppe =N-NH-C(SCH₃)=N-R₆ oder =N-N=C(SCH₃)-NH-R₆ bilden, **dadurch gekennzeichnet**, daß es die Kondensation von H₂N-NH-C(SCH₃)=N-R₆ mit Ninhydrin der Formel: 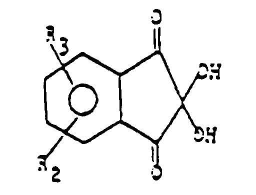 wobei R₂, R₃ und R₆ dieselbe Bedeutung haben wie in Anspruch 1, in der Wärme umfaßt.

14. Verfahren zur Herstellung von Verbindungen der Formel (I), für welche das Paar (A, B) = (N-OH, Sauerstoff) oder (N-OH, N-OH) und R und R₁ zusammen eine Gruppe =N-NH-CX-NH-R₅ oder =N-NH-CX-N(Phenyl)₂ bilden, **dadurch gekennzeichnet**, daß es die Kondensation von einem oder mehreren Äquivalenten Hydroxylamin-Hydrochlorid mit den Verbindungen der Formel (I), gemäß Anspruch 1, für welche das Paar (A, B) = (Sauerstoff, Sauerstoff) und R und R₁ dieselbe Bedeutung wie oben haben, in der Wärme umfaßt.

15. Verfahren zur Herstellung von Verbindungen der Formel (I) gemäß Anspruch 1, für welche das Paar (A, B) = (N-NH-CX-NH-R₅, Sauerstoff), und R und R₁ zusammen eine Gruppe =N-NH-CX-NH-R₅ bilden, **dadurch gekennzeichnet**, daß es die Kondensation von zwei Äquivalenten von H₂N-NH-CX-NH-R₅ mit Ninhydrin der Formel: 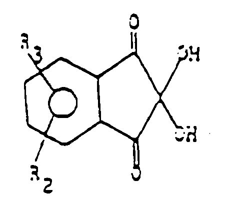 umfaßt, wobei R₂, R₃ und R₅ dieselbe Bedeutung haben wie in Anspruch 1.
